# EUROPEAN PATENT SPECIFICATION

(11) **EP 2 026 775 B1**
(45) Date of publication and mention of the grant of the patent: **29.04.2015**
(21) Application number: 07804842.8
(22) Date of filing: 09.05.2007
(51) Int. Cl.: A61K 31/00, A61K 31/165, A61P 35/00, A61P 35/02, A61P 11/06

(54) **USE OF SYK TYROSINE KINASE INHIBITORS FOR THE TREATMENT OF CELL PROLIFERATIVE DISORDERS**
VERWENDUNG VON SYK-TYROSINKINASEHEMMERN ZUR BEHANDLUNG VON ZELLPROLIFERATIVEN ERKRANKUNGEN
UTILISATIONS DES INHIBITEURS DE TYROSINE KINASE SYK POUR LE TRAITEMENT DE TROUBLES CELLULAIRES PROLIFERATIFS

(30) Priority: 09.05.2006 US 799103 P
(43) Date of publication of application: 25.02.2009
(73) Proprietor: Novaremed Ltd., 4917001 Petah Tiqwa (IL)
(72) Inventor: KAPLAN, Eliahu, 47228 Ramat-HaSharon (IL)
(74) Representative: Mintz Levin Cohn Ferris Glovsky and Popeo LLP
(86) International application number: PCT/IB2007/002471
(87) International publication number: WO 2007/129226

(56) References cited:
- WO-A-2004/031129
- WO-A-2005/092305
- WO-A1-03/000688
- NESTERENKO VITALIY ET AL: "Identification from a combinatorial library of a small molecule that selectively induces apoptosis in cancer cells." JOURNAL OF THE AMERICAN CHEMICAL SOCIETY, vol. 125, no. 48, 3 December 2003 (2003-12-03), pages 14672-14673, XP002468543 ISSN: 0002-7863
- PARK JAE B ET AL: "N-Caffeoyltyramine arrests growth of U937 and Jurkat cells by inhibiting protein tyrosine phosphorylation and inducing caspase-3." CANCER LETTERS, vol. 202, no. 2, 30 December 2003 (2003-12-30), pages 161-171, XP002468544 ISSN: 0304-3835
- LIN LIE-CHWEN ET AL: "Anti-inflammatory neolignans from Piper kadsura." JOURNAL OF NATURAL PRODUCTS MAY 2006, vol. 69, no. 5, 3 May 2006 (2006-05-03), pages 842-844, XP002468545 ISSN: 0163-3864
- AZZOUZ R ET AL: "Selective tetrahydropyranylation under non-acidic conditions" SYNLETT 03 NOV 2005 GERMANY, no. 18, 3 November 2005 (2005-11-03), pages 2808-2810, XP002468546 ISSN: 0936-5214
- GREENFIELD A A ET AL: "Convenient synthesis of functionalized terphenyls" TETRAHEDRON LETTERS, ELSEVIER, AMSTERDAM, NL, vol. 44, no. 13, 24 March 2003 (2003-03-24), pages 2729-2732, XP004413304 ISSN: 0040-4039
- MORISAKI K ET AL: "Synthesis of novel vitamin C phosphodiesters: Stability and antioxidant activity" CARBOHYDRATE RESEARCH, ELSEVIER SCIENTIFIC PUBLISHING COMPANY. AMSTERDAM, NL, vol. 286, 5 June 1996 (1996-06-05), pages 123-138, XP004018659 ISSN: 0008-6215
- AMAT M ET AL: "Synthesis of enantiopure trans-3,4-disubstituted piperidines. An enantiodivergent synthesis of (+)- and (-)-paroxetine." THE JOURNAL OF ORGANIC CHEMISTRY 19 MAY 2000, vol. 65, no. 10, 19 May 2000 (2000-05-19), pages 3074-3084, XP002468547 ISSN: 0022-3263
- NICOLAUS B J R: "SYMBIOTIC APPROACH TO DRUG DESIGN", DECISION MAKING IN DRUG RESEARCH, XX, XX, 1 January 1983 (1983-01-01), pages 173-186, XP001111439,

## Description

The invention relates to compounds and their use in treating cell proliferative disorders, more specifically colon cancer.

Protein tyrosine kinases (PTKs) are enzymes which catalyze the phosphorylation of tyrosine residues. There are two main classes of PTKs: receptor PTKs and cellular, or non-receptor, PTKs. These enzymes are involved in cellular signaling pathways and regulate key cell functions such as proliferation, differentiation, anti-apoptotic signaling and neurite outgrowth. Unregulated activation of these enzymes, through mechanisms such as point mutations or over-expression, can lead to various forms of cancer as well as benign proliferative conditions.

More than 70% of the known oncogenes and proto-oncogenes involved in cancer code for PTKs. The importance of PTKs in health and disease is further underscored by the existence of aberrations in PTK signaling occurring in inflammatory diseases and diabetes.

Receptor PTKs possess an extracellular ligand binding domain, a transmembrane domain and an intracellular catalytic domain. The transmembrane domain anchors the receptor in the plasma membrane, while the extracellular domains bind growth factors. Characteristically, the extracellular domains are comprised of one or more identifiable structural motifs, including cysteine-rich regions, fibronectin III-like domains, immunoglobulin-like domains, EGF-like domains, cadherin-like domains, kringle-like domains, Factor VIII-like domains, glycine-rich regions, leucine-rich regions, acidic regions and discoidin-like domains.

The intracellular kinase domains of receptor PTKs can be divided into two classes: those containing a stretch of amino acids separating the kinase domain and those in which the kinase domain is continuous. Activation of the kinase is achieved by ligand binding to the extracellular domain, which induces dimerization of the receptors. Receptors activated in this way are able to autophosphorylate tyrosine residues outside the catalytic domain via cross-phosphorylation. The results of this auto-phosphorylation are stabilization of the active receptor conformation and the creation of phosphotyrosine docking sites for proteins which transduce signals within the cell. Signaling proteins which bind to the intracellular domain of receptor tyrosine kinases in a phosphotyrosine-dependent manner include RasGAP, PI3-kinase, phospholipase C _{y}, phosphotyrosine phosphatase SHP and adaptor proteins such as Shc, Grb2 and Crk.

In contrast to receptor PTKs, cellular PTKs are located in the cytoplasm, nucleus or anchored to the inner leaflet of the plasma membrane. They are grouped into eight families: SRC, JAK, ABL, FAK, FPS, CSK, SYK and BTK. With the exception of homologous kinase domains (Src Homology 1, or SH1 domains), and some protein-protein interaction domains (SH2 and SH3 domains), they have little in common, structurally. Of those cellular PTKs whose functions are known, many, such as SRC, are involved in cell growth. In contrast, FPS PTKs are involved in differentiation, ABL PTKs are involved in growth inhibition, and FAK activity is associated with cell adhesion. Some members of the cytokine receptor pathway interact with JAKs, which phosphorylate the transcription factors, STATs. Still other PTKs activate pathways whose components and functions remain to be determined.

Syk is a non-receptor tyrosine kinase related to ZAP-70 that is involved in signaling from the B-cell receptor and the IgE receptor. Syk binds to ITAM motifs within these receptors, and initiates signaling through the Ras, PI 3-kinase, and PLCg signaling pathways. Syk is also widely expressed in hematopoietic cells. It is involved in coupling activated immunoreceptors to downstream signaling events that mediate diverse cellular responses including proliferation, differentiation and phagocytosis. Syk expression has been reported in cell lines of epithelial origin, but its function in these cells remains unknown. Syk is commonly expressed in normal human breast tissue, benign breast lesions and low-tumorigenic breast cancer cell lines. Syk is believed to be a potent modulator of epithelial cell growth and a potential tumor suppressor in human breast carcinomas.

ZAP-70 is a non-receptor tyrosine kinase of the Syk family. ZAP-70 is involved in T-cell receptor signaling through its recruitment to T-cell receptor complexes once T-cells are activated by antigen presenting cells. ZAP-70, once activated, phosphorylates and activates several downstream targets, including SLP-76 and LAT. ZAP-70 has been identified as the newest biomarker for Chronic Lymphocytic Leukemia (CLL) prognosis.

WO 2005/092305 relates to compounds for use in the treatment of viral diseases and infections. WO 03/000688 relates to compounds for use in the treatment of colon cancer.

There is a need in the art to develop potent compounds that are effective in treating, preventing and controlling cell proliferative disorders, and more specifically Syk tyrosine kinase-mediated disorders.

The scope of the invention is defined by the claims.

According to one aspect of the present invention therefore there is provided a compound for use in treating a cell proliferative disorder in a subject, said compound being the *S-*enantiomer of a compound represented by the structure of formula I: 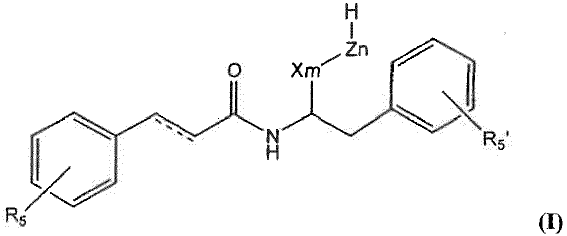 wherein:
the dotted line represents a single or a double bond; and R₅ and R₅' are independently H, OH or OR₆, where R₆ is a linear or branched C₁-C₄ alkyl ; X is -CH₂O, -CH₂CH₂O, -CH(CH₃)CH₂O, or -CH₂CH(CH₃)O; Z is -CH₂CH₂O,-CH(CH₃)CH₂O, or -CH₂CH(CH₃)O; m is 1; and n is an integer of 1-50, or a pharmaceutically effective salt thereof, wherein the cell proliferative disorder is colon cancer.

According to yet another aspect of the present invention, there is provided the use of an S-enantiomer of compound represented by the structure of formula I defined above in the manufacture of a medicament for treating a cell proliferative disorder, comprising colon cancer.

In some embodiments, n may be an integer from 1-25. For example, n may be 1, 2, 3, 4, 5, 6, 7, 8, 9, 10, 11 12, 13, 14, 15, 16, 17, 18, 19, 20, 21, 22, 23, 24 or 25. Preferably, n is 1, 2 or 7.

In another embodiment, the invention comprehends the use of the salts or hydrates of the compound of formula I. In one embodiment, X may be -CH₂O.

In addition, the invention provides a compound for use in treating a cell proliferative disorder comprising colon cancer, using an *S*-enantiomer of a compound represented by the structure of formula II: 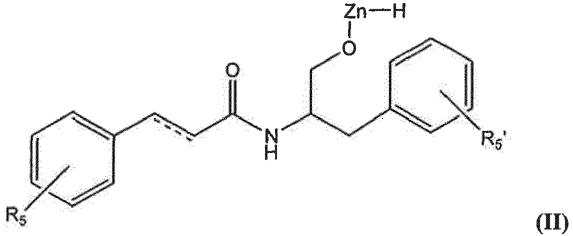 where the dotted line represents a single or a double bond; R₅ and R₅' are independently H, OH, or OR₆ (where R₆ is a linear or branched C₁-C₄ alkyl); Z is - CH₂CH₂O, -CH(CH₃)CH₂O, or -CH₂CH(CH₃)O; and n is an integer of 1-25. For example, n can be 1, 2, 3, 4, 5, 6, 7, 8, 9, 10, 11, 12, 13, 14, 15, 16, 17, 18, 19, 20, 21, 22, 23, 24 or 25. Preferably, n is 1, 2 or 7.In another embodiment, the invention provides the salts or hydrates of the compound presented by the structure of formula II. In one embodiment, Z is -CH(CH₃)CH₂O.

In addition, the invention provides a compound for use in treating a cell proliferative disorder comprising colon cancer, using an *S*-enantiomer of a compound represented by the structure of formula III: 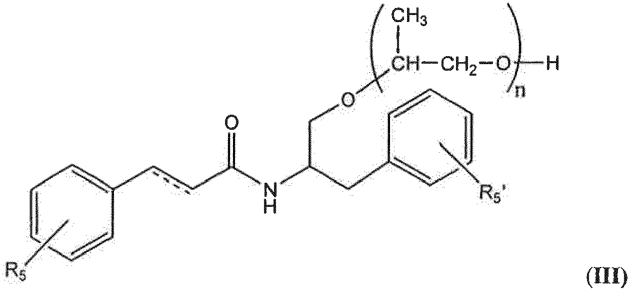 where the dotted line represents a single or a double bond; R₅ and R₅' are independently H, OH or OR₆ (where R₆ is a linear or branched C₁-C₄ alkyl); Z is - CH₂CH₂O, -CH(CH₃)CH₂O, or -CH₂CH(CH₃)O; and n is an integer of 1-25. For example, n can be 1, 2, 3, 4, 5, 6, 7, 8, 9, 10, 11, 12, 13, 14, 15, 16, 17, 18, 19, 20, 21, 22, 23, 24 or 25. Preferably, n is 1, 2 or 7.

In another embodiment, the invention provides the salts or hydrates of the compound presented by the structure of formula III. In one embodiment, R₅ is H. In another embodiment, R₅ is OH. In one embodiment, R₅' is H. In another embodiment, R₅' is OH.

In addition, the invention provides a compound for use in treating a cell proliferative disorder comprising colon cancer, using an *S*-enantiomer of a compound of Formula IV: 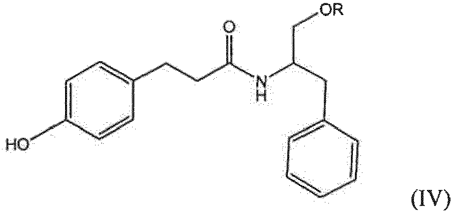 where R is a polyalkylene glycol polymer having n units, n is an integer from 1-50. In one embodiment, the polyalkylene glycol polymer is polyisopropylene glycol. In another embodiment, n is an integer from 1-25. For example, n can be 1, 2, 3, 4, 5, 6, 7, 8, 9, 10, 11, 12, 13, 14, 15, 16, 17, 18, 19, 20, 21, 22, 23, 24 or 25. Preferably, n is 1, 2 or 7.

In addition, the invention provides a compound for use in treating a cell proliferative disorder comprising colon cancer, using an *S*-enantiomer of a compound of Formula V: 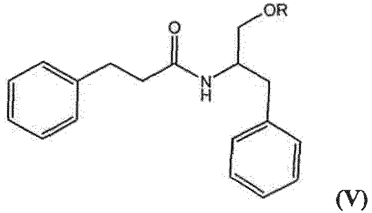 where R is a polyalkylene glycol polymer having n units, n being an integer from 1-50. In one embodiment, the polyalkylene glycol polymer is polyisopropylene glycol. n can be, *e.g.,* 1, 2, 3, 4, 5, 6, 7, 8, 9, 10, 11, 12, 13, 14, 15, 16, 17, 18, 19, 20, 21, 22, 23, 24 or 25. Preferably, n is 1, 2 or 7.

In addition, the invention provides a compound for use in treating a cell proliferative disorder comprising colon cancer, using an *S*-enantiomer of a compound of Formula VI: 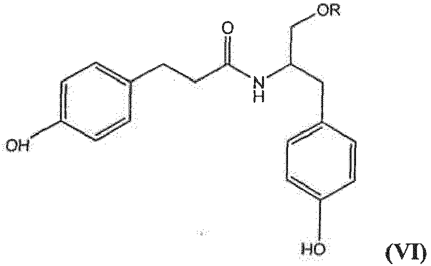 where R is a polyalkylene glycol polymer having n units, n being an integer from 1-50. In one embodiment, the polyalkylene glycol polymer is polyisopropylene glycol. n can be, *e.g.,* 1, 2, 3, 4, 5, 6, 7, 8, 9, 10, 11, 12, 13, 14, 15, 16, 17, 18, 19, 20, 21, 22, 23, 24 or 25. Preferably, n is 1, 2 or 7.

In addition, the invention provides a compound for use in treating a cell proliferative disorder, using an *S*-enantiomer of a compound of Formula VII: 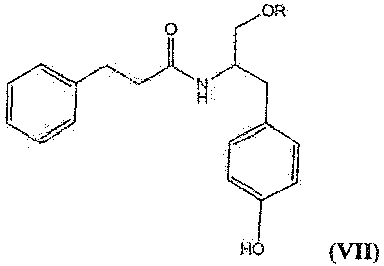 where R is a polyalkylene glycol polymer having n units, n being an integer from 1-50. In one embodiment, the polyalkylene glycol polymer is polyisopropylene glycol. n can be, *e.g.,* 1, 2, 3, 4, 5, 6, 7, 8, 9, 10, 11, 12, 13, 14, 15, 16, 17, 18, 19, 20, 21, 22, 23, 24 or 25. Preferably, n is 1, 2 or 7.

Furthermore, in one embodiment, the invention provides a composition for use in treating a cell proliferative disorder, comprising colon cancer, comprising one or more compounds of formula I, II, III, IV, V, VI or VII. In another embodiment, the invention provides a pharmaceutical composition for use in treating colon cancer comprising as an active ingredient one or more compounds of formula I, II, III, IV, V, VI or VII, together with one or more pharmaceutically acceptable excipients or adjuvants.

Unless otherwise defined, all technical and scientific terms used herein have the same meaning as commonly understood by one of ordinary skill in the art to which this invention belongs. Suitable methods and materials are described below.

In addition, the materials, methods, and examples are illustrative only.

All parts and percentages are by weight unless otherwise specified.

### Definitions

For convenience, certain terms used in the specification, examples, and appended claims are collected here.

"Treating", includes any effect, e.g., lessening, reducing, modulating, or eliminating, that results in the improvement of the condition, disease, disorder, etc.

The invention provides compounds and pharmaceutical compositions useful in the treatment of colon cancer. The invention further provides one or more of the compounds represented by the structure of formula I, II, III, IV, V, VI or VII above for use in the treatment, prevention and control of colon cancer.

The synthetic methodologies for obtaining and purifying the compounds are disclosed in detail below. However, it should be apparent to a person skilled in the art that the compounds of the invention can be prepared by any feasible synthetic method and that the syntheses set forth herein are in no way limiting. Various synthetic methods for the preparation and purification of these compounds will be known to a person skilled in the art. Compounds of the invention may be further modified as allowed by the rules of chemistry. Such modifications include the addition of various substituents (e.g., hydroxylation, carboxylation, methylation, etc.), generation of enantiomers, creation of acid- or base-addition salts, and the like. Other modifications include adding polyalkylene glycol polymers.

The compounds of the invention may be synthesized as polyalkylene glycol (PAG) conjugates. Typical polymers used for conjugation include poly(ethylene glycol) (PEG), also known as or poly(ethylene oxide) (PEO) and polypropylene glycol (including poly isopropylene glycol). These conjugates are often used to enhance solubility and stability and to prolong the blood circulation half-life of molecules.

In its most common form, a polyalkylene glycol (PAG), such as PEG is a linear polymer terminated at each end with hydroxyl groups:

HO-CH₂CH₂O-(CH₂CH₂O)ₙ-CH₂CH₂-OH.

The above polymer, alpha-, omega-dihydroxylpoly(ethylene glycol), can also be represented as HO-PEG-OH, where it is understood that the -PEG-symbol represents the following structural unit:

-CH₂CH₂O-(CH₂CH₂O)ₙ-CH₂CH₂-

where n typically ranges from about 4 to about 10,000. PEG is commonly used as methoxy-PEG-OH, or mPEG, in which one terminus is the relatively inert methoxy group, while the other terminus is a hydroxyl group that is subject to ready chemical modification. Additionally, random or block copolymers of different alkylene oxides (e.g., ethylene oxide and propylene oxide) that are closely related to PEG in their chemistry can be substituted for PEG in many of its applications.

PAGs are polymers which typically have the properties of solubility in water and in many organic solvents, lack of toxicity, and lack of immunogenicity. One use of PAGs is to covalently attach the polymer to insoluble molecules to make the resulting PAG-molecule "conjugate" soluble. For example, it has been shown that the water-insoluble drug paclitaxel, when coupled to PEG, becomes water-soluble. Greenwald, et al., J. Org. Chem., 60:331-336 (1995).

Polyalkylated compounds typically contain between 1 and 500 monomeric units. Other PAG compounds contains between 1 and 200 monomeric units. Still other PAG compounds contain between 1 and 100 monomeric units. For example, the polymer may contain 1, 10, 20, 30, 40, 50, 55, 60, 65, 70, 75, 80, 85, 90, 95, or 100 monomeric units. Some compounds contain polymers which include between 5 and 75 or between 1 and 50 monomeric units. For example, the polymer may contain 2, 3, 5, 6, 7, 8, 10, 11, 12, 13, 15, 16, 17, 18, 20, 25, 30, 33, 34, 35, 40, 45, 50, 60, 65, 68, 69, 70, or 75 monomeric units. Preferably, n is 7, 12, 17, 34 or 69. The polymers can be linear or branched.

It is to be understood that compounds which have been modified by the addition of a PAG moiety may include a mixture of polymers which have a varying number of monomeric units. Typically, the synthesis of a PAG-modified compound (e.g., a PAG-conjugate) will produce a population of molecules with a Poisson distribution of the number of monomeric units per polymer in the conjugate. Thus, a compound described as having a polymer of n = 7 monomeric units refers not only to the actual polymers in that population being described as having n = 7 monomeric units, but also to a population of molecules with the peak of the distribution being 7. The distribution of monomeric units in a given population can be determined, e.g., by nuclear magnetic resonance (NMR) or by mass spectrometry (MS).

Throughout this application, conventional terminology is used to designate the isomers as described below and in appropriate text books known to those of ordinary skill in the art. *(see, e.g.,* Principles in Biochemistry, Lehninger (ed.), page 99-100, Worth Publishers, Inc. (1982) New York, NY; Organic Chemistry, Morrison and Boyd, 3rd Edition, Chap. 4, Allyn and Bacon, Inc., Boston, MA (1978).

It will be noted that the structure of the compounds of the invention includes asymmetric carbon atoms. It is to be understood accordingly that the isomers arising from such asymmetry (e.g., all enantiomers and diastereomers) are included within the scope of the invention, unless indicated otherwise. Such isomers can be obtained in substantially pure form by classical separation techniques and by stereochemically controlled synthesis. Furthermore, the structures and other compounds and moieties discussed in this application also include all tautomers thereof. Alkenes can include either the E- or Z-geometry, where appropriate.

A carbon atom which contains four different substituents is referred to as a chiral center. A chiral center can occur in two different isomeric forms. These forms are identical in all chemical and physical properties with one exception, the direction in which they can cause the rotation of plane-polarized light. These compounds are referred to as being "optically active," *i.e.,* the compounds can rotate the plane-polarized light in one direction or the other.

The four different substituent groups attached to a carbon can occupy two different arrangements in space. These arrangements are not superimposable mirror images of each other and are referred to as optical isomers, enantiomers, or stereoisomers. A solution of one stereoisomer of a given compound will rotate plane polarized light to the left and is called the levorotatory isomer [designated (-)]; the other stereoisomer for the compound will rotate plane polarized light to the same extent but to the right and is called dextrorotatory isomer [designated (+)].

The R S system was invented to avoid ambiguities when a compound contains two or more chiral centers. In general, the system is designed to rank the four different substituent atoms around an asymmetric carbon atom in order of decreasing atomic number or in order of decreasing valance density when the smallest or lowest-rank group is pointing directly away from the viewer. If the decreasing rank order is seen to be clockwise, the configuration around the chiral center is referred to as R; if the decreasing rank order is counter-clockwise, the configuration is referred to as S. Each chiral center is named accordingly using this system.

A compound of the invention may be prepared by asymmetric synthesis, or by derivation with a chiral auxiliary, where the resulting diastereomeric mixture is separated and the auxiliary group cleaved to provide the pure desired S- enantiomer. Alternatively, where the molecule contains a basic functional group, such as amino, or an acidic functional group, such as carboxyl, diastereomeric salts are formed with an appropriate optically-active acid or base, followed by resolution of the diastereomers thus formed by fractional crystallization or chromatographic means well known in the art, and subsequent recovery of the pure enantiomer.

The compositions and pharmaceutical compositions of the invention may comprise one or more of the compounds of the invention, either in a pure form or a partially pure form. Similarly, the methods comprise using one or more compounds, wherein the compounds are in a pure form, a partially pure form. Preferably, said pharmaceutical composition may comprise at least 95% by weight of said one or more compounds of the invention, e.g., 96%, 97%, 98% or more than 99% by weight of said one more compounds.

In some embodiments, the pharmaceutical composition in accordance with the present invention may include, in addition to the One or more compounds of the invention, a proportion of free polyalkylene glycol such, for example, as polyethylene glycol (PEG) or polypropylene glycol (PPG). Said polyalkylene glycol may itself be biologically active. The chain length of the polyalkylene glycol may range from 1-50, especially 1-25. In some embodiments, said polyalkylene glycol may have a chain length of 3, 7, 12 and/or 17 monomeric units. Typically, said pharmaceutical composition may comprise an amount of free polyethylene glycol and/or polypropylene glycol having a mixture of different chain lengths. In some embodiments, said pharmaceutical composition may comprise about 5-60% by weight of one or more compounds according to the invention and about 95-40% by weight of free polyalkylene glycol. Typically, said pharmaceutical composition may comprise about 45-55% by weight of said one or more compounds according to the invention and about 55-45% weight said one or more polyalkylene glycols. Alternatively, said pharmaceutical composition may comprise about 80-95% by weight of said one or more compounds according to the invention and about 20-5% by weight of said one or more polyalkylene glycols.

In one embodiment, a composition of the invention comprises at least one of the compounds of the invention, *i.e.* one or more of the compounds represented by the structures of formula I, II, III, IV, V, VI or VII. In another embodiment, a composition of the invention comprises a mixture of at least two of the compounds represented by the structures of formula I, II, III, IV, V, VI or VII. In another embodiment, a composition of the invention comprises a mixture of at least five of the compounds represented by the structures of formula I, II, III, IV, V, VI or VII. In another embodiment, a composition of the invention comprises a mixture of at least ten of the compounds represented by the structures of formula I, II, III, IV, V, VI or VII.

It has now been surprisingly found that one or more compounds represented by the structures of formula I, II, III, IV, V, VI or VII are effective against colon cancer. Thus, in one embodiment, the invention provides compounds for the treatment, prevention and control of colon cancer in human as well as veterinary applications. In one embodiment, the treatment comprises administering to a subject one or more compounds represented by the structures of formula I, II, III, IV, V, VI or VII. In another embodiment, the treatment comprises administering to a subject a pharmaceutical composition comprising one or more compounds represented by the structures of formula I, II, III, IV, V, VI or VII.

Methods of administration are well known to a person skilled in the art. Methods of administration include parenterally, transdermally, intramuscularly, intravenously, intradermally, intranasally, subcutaneously, intraperitoneally, or intraventricularly or rectally. Methods and means of administration are known to those skilled in the art, for example, U.S. Patent Nos. 5,693,622; 5,589,466; 5,580,859; and 5,566,064.

In addition, the invention provides a pharmaceutical composition comprising as an active ingredient one or more compounds of the invention, together with one or more pharmaceutically acceptable excipients. As used herein, "pharmaceutical composition" can mean a therapeutically effective amount of one or more compounds of the invention together with suitable excipients and/or carriers useful for the treatment of colon cancer.

A "therapeutically effective amount" as used herein refers to that amount that provides a therapeutic effect for a given condition and administration regimen. Such compositions can be administered by any one of the methods listed hereinabove.

A further aspect comprises a compound in combination with other compounds of the invention. A compound may also be administered in combination with an anti-inflammatory agent, an immunosuppressant, an antiviral agent, or the like. Furthermore, the compounds may be administered in combination with a chemotherapeutic agent such as an alkylating agent, anti-metabolite, mitotic inhibitor or cytotoxic antibiotic, as described above. In general, the currently available dosage forms of the known therapeutic agents for use in such combinations will be suitable.

Combination therapy" (or "co-therapy") includes the administration of a compound and at least a second agent as part of a specific treatment regimen intended to provide the beneficial effect from the co-action of these therapeutic agents. The beneficial effect of the combination includes, pharmacokinetic or pharmacodynamic co-action resulting from the combination of therapeutic agents. Administration of these therapeutic agents in combination typically is carried out over a defined time period (usually minutes, hours, days or weeks depending upon the combination selected). "Combination therapy" may, but generally is not, intended to encompass the administration of two or more of these therapeutic agents as part of separate monotherapy regimens that incidentally and arbitrarily result in the combinations of the invention.

"Combination therapy" is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single capsule having a fixed ratio of each therapeutic agent or in multiple, single capsules for each of the therapeutic agents.

Sequential or substantially simultaneous administration of each therapeutic agent can be effected by any appropriate route including, oral routes, intravenous routes, intramuscular routes, and direct absorption through mucous membrane tissues. The therapeutic agents can be administered by the same route or by different routes. For example, a first therapeutic agent of the combination selected may be administered by intravenous injection while the other therapeutic agents of the combination may be administered orally. Alternatively, for example, all therapeutic agents may be administered orally or all therapeutic agents may be administered by intravenous injection. The sequence in which the therapeutic agents are administered is not narrowly critical.

"Combination therapy" also can embrace the administration of the therapeutic agents as described above in further combination with other biologically active ingredients and non-drug therapies (*e.g.,* surgery or radiation treatment.) Where the combination therapy further comprises a non-drug treatment, the non-drug treatment may be conducted at any suitable time so long as a beneficial effect from the co-action of the combination of the therapeutic agents and non-drug treatment is achieved. For example, in appropriate cases, the beneficial effect is still achieved when the non-drug treatment is temporally removed from the administration of the therapeutic agents, perhaps by days or even weeks.

The compounds and the other pharmacologically active agent may be administered to a patient simultaneously, sequentially or in combination. It will be appreciated that when using a combination the compound and the other pharmacologically active agent may be in the same pharmaceutically acceptable carrier and therefore administered simultaneously. They may be in separate pharmaceutical carriers such as conventional oral dosage forms which are taken simultaneously. The term "combination" further refers to the case where the compounds are provided in separate dosage forms and are administered sequentially.

The compounds may optionally be administered in a combination therapy with other tyrosine kinase inhibitors, e.g., for cancer treatment, including trastuzumab (HERCEPTIN), gefitinib (IRESSA), erlotinib (TARCEVA) and lapatinib. The tyrosine kinase inhibitor may be active on Syk or not.

The compositions and combination therapies may be administered in combination with a variety of pharmaceutical excipients, including stabilizing agents, carriers and/or encapsulation formulations as described herein.

In one embodiment, the compositions are formulated as oral or parenteral dosage forms, such as uncoated tablets, coated tablets, pills, capsules, powders, granulates, dispersions or suspensions. In another embodiment, the compositions are formulated for intravenous administration. In another embodiment, the compounds of the invention are formulated in ointment, cream or gel form for transdermal administration. In another embodiment, the compounds are formulated as an aerosol or spray for nasal application. In another embodiment, the compositions are formulated in a liquid dosage form. Examples of suitable liquid dosage forms include solutions or suspensions in water, pharmaceutically acceptable fats and oils, alcohols or other organic solvents, including esters, emulsions, syrups or elixirs, solutions and/or suspensions.

Suitable excipients and carriers can be solid or liquid and the type is generally chosen based on the type of administration being used. Liposomes may also be used to deliver the composition. Examples of suitable solid carriers include lactose, sucrose, gelatin and agar. Oral dosage forms may contain suitable binders, lubricants, diluents, disintegrating agents, coloring agents, flavoring agents, flow-inducing agents, and melting agents. Liquid dosage forms may contain, for example, suitable solvents, preservatives, emulsifying agents, suspending agents, diluents, sweeteners, thickeners, and melting agents. Parenteral and intravenous forms should also include minerals and other materials to make them compatible with the type of injection or delivery system chosen.

Following is a description by way of example only with reference to the accompanying drawings of embodiments of the present invention.

In the drawings:
**FIG 1** illustrates the inhibitory effect of treating PHA-activated PBMCs with varying concentrations of a compound of the invention, AV 61S in a DMSO carrier.
**FIG 2** illustrates the inhibitory effect of treating PHA-activated PBMCs with varying concentrations of a compound of the invention, AV 61S in a PBS carrier;
**FIG 3** illustrates the lack of effect of AV 61S has on E6.1 Jurkat cell survival after a seven day period;
**FIG 4** illustrates the effect of AV 61 S and AV 74S on Jurkat cell survival after a seven day period;
**FIG 5** illustrates the effect of treating PHA-activated PBMCs with varying concentrations of a compound of the invention, AV 61S;
**FIG 6** illustrates the effect of treating PHA-activated PBMCs with varying concentrations of AV 61S;
**FIG 7** illustrates the effect of treating PHA-activated PBMCs with varying concentrations of a compound of the invention, AV 74S;
**FIG 8** illustrates the effect of treating PHA-activated PBMCs with varying concentrations of AV 74S;
**FIG 9** illustrates a dose response curve for compound AV 61S on activated PBMCs;
**FIG 10** illustrates a dose response curve for compound AV 74S on activated PBMCs;
**FIG 11** illustrates the comparative effect of AV 61 S and AV 74S treatment on T and B cells compared to their R forms;
**FIG 12** illustrates the effect of AV 74S treatment on monocytes;
**FIG 13** illustrates the lack of inhibitory effect of PPG on Jurkat cells after two days (an amount of unreacted PPG is possibly present after compound synthesis);
**FIG 14** illustrates the lack of inhibitory effect of PPG on Jurkat cells after four days;
**FIG 15** illustrates, in comparison with cyclosporin-treated PBMCs, the viability of PBMCs treated with differing concentrations of AV 61S or AV 74S after 24 hours;
**FIG 16** illustrates, in comparison with cyclosporin-treated PBMCs, the viability of PBMCs treated with differing concentrations of AV 61S or AV 74S after 48 hours;
**FIG 17** illustrates, in comparison with cyclosporin-treated PBMCs, the viability of PBMCs treated with differing concentrations of AV 61 S or AV 74S after 72 hours, and of combined dosages of AV 61S and AV 74S at different concentrations;
**FIG 18** illustrates, in comparison with cyclosporin-treated Jurkat cells, the viability of E6.1 Jurkat cells treated with differing concentrations of AV 61S or AV 74S after 24 hours; and
**FIG 19** illustrates, in comparison with cyclosporin-treated Jurkat cells, the viability of E6.1 Jurkat cells treated with differing concentrations of AV 61S or AV 74S after 48 hours.
**FIGS 20A-I** illustrates the dose response curves for AV 74S when tested against nine different panels of cell lines.
**FIG 21** shows the dose response curves for AV 74S for all of the cell lines of **FIGs 20A-I****.**

### DESCRIPTION 1: Synthesis of Polvalkylene Glycol Compounds

Polyalkylene glycol compounds were generally synthesized by preparation of the appropriate alcohol compound (e.g., one of the compounds described in Example 1, or a hydroxylated derivative thereof) and then conjugation of the alcohol with a polyalkylene glycol (PAG) polymer (e.g., polyethylene glycol (PEG) or polypropylene glycol (PPG)) of the desired length.

### Compound 1, Phenyl alaninol

1.2 gr, 32 mM, of LiAlH₄ were added to 2.3 gr, 10 mM, phenyl alanine ethyl ester HCl in 50 ml dry ether. After stirring for 2 hours at room temperature, water and KOH were added and the reaction product was extracted with ethyl acetate. After evaporation, 0.8 gr of compound 1, a light yellow oil, was obtained.

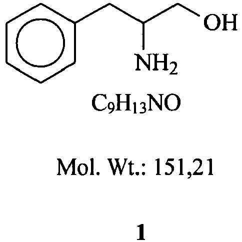

Compound 1 crystallized on standing. Mp-70. NMR CDCl₃ 7.30(5H,m), 3.64(1H,dd,J=10.5,3.8 Hz) 3.40(1H,dd,J=10.5,7.2 Hz) 3.12(1H,m), 2.81(1H,dd,J=13.2, 5.2 Hz), 2.52(1H, dd, J=13.2, 8.6 Hz) NMR acetone d₆ 7.30(5H, m), 3.76(1H, dt) 3.60(1H, m) 3.30 (1H, t),2.85(2H, m). Helv. Chim. Acta, 31, 1617(1948). Biels. -E3,Vol. 13,p 1757.

### Compound 2, Tyrosinol

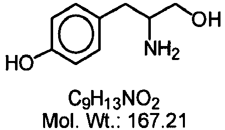

To 3 gr, 12 mM, L- tyrosine ethyl ester HCl in 50 ml dry ether was added 1.2 gr 32 mM LiAlH₄. After stirring 3 hours at room temperature, water and KOH were added and the reaction was extracted with ethyl acetate. Evaporation gave 1.1 gr of a light yellow oil, 54% yield, which on standing crystallized. mp-85.

NMR CDCl₃ 7.20(4H,AB q,J=8.6 Hz), 3.50(2H,m) 3.20(1H,m), 2.81(2H,m). NMR tyrosine ethyl ester free base CDCl₃ 7.0,6.56(4H, AB q, J=8.8 Hz), 4.20(2H, q, J=7, 0 Hz), 3.70, 3.0, 2.80(3H, 12 line ABXm), 1.28. (3H, t, J=7.0 Hz). JACS 71 305(1949). Biels. -E3, Vol. 13, p 2263.

### Compound 3, Tryptophanol

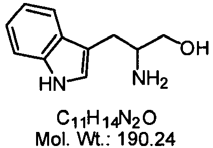

To 3 gr, 12.9 mM, L-tryptophan methyl ester HCl in 50 ml dry ether was added 1.2 gr,32 mM LiAlH₄.. After stirring 6 hours at room temperature water and KOH were added and the reaction extracted with ethyl acetate. Evaporation gave 1.23 gr light yellow oil, 50% yield. On standing crystallized. Mp-65..

NMR CDCl₃ 7.30(5H, m), 3.64(1H, dd, J=10.5, 3.8 Hz) 3.40(1H, dd, J=10.5, 7.2 Hz) 3.12 (1H, m), 2.81(1H, dd, J=13.2, 5.2 Hz), 2.52(1H, dd, J=13.2, 8.6 Hz) J. Het. Chem, 13, 777 (1976). Biels. -E5, 22,Vol. 12, p 90.

### Compound 4, AV 22

0.66 gr 4-hydroxy hydrocinnamic acid and 4 ml thionyl chloride in 30 ml cyclohexane were refluxed for 2 hours. After evaporation, a white solid was obtained, to which 0.65 gr oil of **Compound 1** (4.3 mM) in 30 ml dichloromethane and 0.4 ml triethyl amine were added. After stirring for 2 hours at room temperature, water and KOH were added in order to neutralize the pH. The reaction product was extracted with dichloromethane. Evaporation gave 0.8 gr of compound 4, light yellow viscous oil. Part of this product was triturated and recrystallized with ethanol to give a white solid. Mp-149.

NMR CDCl₃ 7.30-6.9(9H, m), 3.50(2H, m) 3.30(2H, t, J=7.2 Hz) 2.90 (3H, m), 2.60(2H, t, J=7.2 Hz).

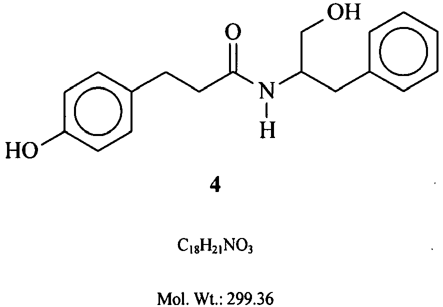

### Compound 5, AV 57

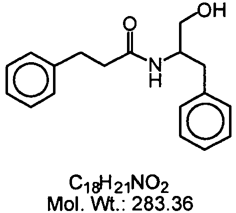

0.75 gr, 5mM, hydrocinnamic acid and 4 ml thionyl chloride in 30 ml cyclohexane were refluxed for 2 hours. Evaporation gave a white solid to which were added 0.83 gr, 5.5 mM, phenyl alaninol in 30 ml dichloromethane and 0.5 ml triethyl amine. After stirring 3 hours at room temperature, water and KOH were added to neutral pH and the reaction was extracted with dichloromethane. Evaporation gave 0.57 gr of a yellow viscous oil, 40% yield.

NMR CDCl₃ 7.40-7.10(10H, m), 3.60(2H, m) 3.35(2H, t, J=7.2 Hz) 2.95 (3H, m), 2.50(2H, t, J=7.2 Hz).

### Compound 6, AV 58

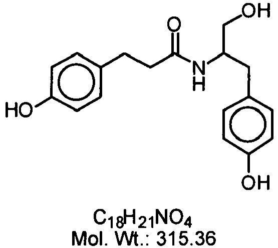

0.66 gr, 4mM, 4-hydroxy hydrocinnamic acid and 4 ml thionyl chloride in 30 ml cyclohexane were refluxed 3 hours. Evaporation gave a light yellow solid to which were added 0.72 gr, 4.3 mM, tyrosinol in 30 ml dichloromethane and 0.5 ml triethyl amine. After stirring 3 hours at room temperature. water and KOH were added to neutral pH and the reaction was extracted with dichloromethane. Evaporation gave 0.53 gr light yellow viscous oil, 42% yield.

NMR CDCl₃ 7.30, 7.20 (8H, 2 ABq, J=8.6 Hz), 3.40(2H, m) 3.30(2H, t, J=7.2 Hz) 2.90 (3H, m), 2.60(2H, t, J=7.2 Hz).

### Compound 8, AV 72

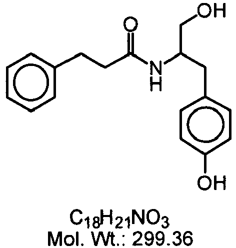

0.45 gr, 3mM, hydrocinnamic acid and 3 ml thionyl chloride in 30 ml cyclohexane were refluxed for 2 hours. Evaporation gave a light yellow solid to which were added 0.58 gr, 3.5 mM, tyrosinol in 30 ml dichloromethane and 0.4 ml triethyl amine. After stirring for 2.5 hours at room temperature, water and KOH were added to attain neutral pH and the reaction was extracted with dichloromethane. Evaporation gave 0.57 gr light yellow viscous oil, 63% yield.

NMR CDCl₃ 7.40-7.10 (9H, m), 3.60(2H, m) 3.35 (2H, t, J=7.2 Hz) 2.95 (3H, m), 2.50 (2H, t, J=7.2 Hz).

### Compound 10

0.3 gr of Compound 4 (AV 22), 0.8 gr, triphenyl phosphine and 0.55 gr ethyl diazo carboxylate were added to 1 gr of poly(propylene glycol), (average molecular weight ca 1000), in 60 ml dichloromethane. Stirring for 2 hours at room temperature, evaporation and chromatography gave 0.65 gr of Compound 10 as a viscous oil.

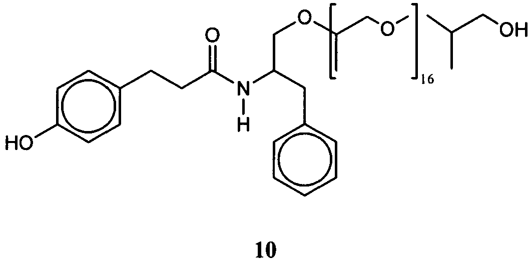

### Compounds synthesized from phenyl alaninol

These compounds include those represented by the structure of formula (VIII):

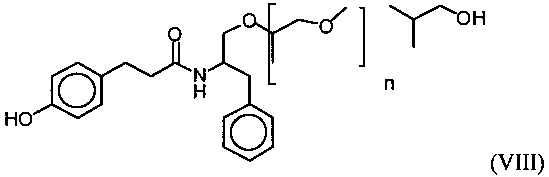

This compound can also be represented as Formula A, where R is a polypropylene glycol polymer and n is the total number of polypropylene monomers in the polymer:

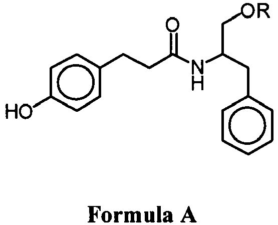

### AV 61S:

R= PPG (polypropylene glycol) n=7 MW-706

0.3 gr **AV 22** (1mM), 0.8 gr, 3 mM, triphenyl phosphine and 0.55 gr 3.2 mM, ethyl diazo carboxylate were added to 1 gr of poly(propylene glycol) (average mol. weight 424 , N=7) in 60 ml dichloromethane. After stirring for 4 hours at room temperature, evaporation and chromatography gave 0.55 gr viscous oil, a 73% yield. NMR CDCl₃ 7.30-6.9(9H, m), 4.1-3.0(m), 2.60(2H, t, J=7.2 Hz), 1.2-1.1(m). 0.1g, 0.33mmol of this product, potassium carbonate (0.069g, 0.5 mmol, thinly crushed) and THF (3 mL, dried over KOH pellets) were put in a round-bottom flask equipped with a magnetic stirrer and a CaCl₂ drying tube. The mixture was cooled over an ice-salt bath (-10°C) and a pre-cooled solution of di-tert-butyldicarbonate (0.066g, 0.30 mmole) in 2 mL THF (dried) was introduced dropwise. The mixture was allowed to stir at ice temperature for 1 hour and then for 2 days at room temperature. The reaction mixture was then evaporated, water (5 mL) introduced and the product was extracted with two 10 mL portions of ethyl acetate. The combined extracts were dried over anhydrous magnesium sulfate, paper-filtered and the solvent removed. The oily residue was triturated with a small amount of n-hexane and the solid formed recovered by vacuum filtration (yield 0.12g, 90.1%). Alternatively, the oily residue can be dissolved in an 1:1 mixture of ethyl acetate and hexane and the product recrystallized.

### AV 62

R= PPG n=12 MW-996

Was prepared as above from 0.2 gr AV 22 to give 0.3 gr, 46% yield.

### AV 60S

R= PPG n=17 MW-1286

Was prepared using the same procedure as **AV 61S,** above, with the substitution of the PPG 7 for PPG 17.

Compounds synthesized from Compound 5, AV 57

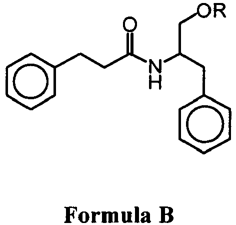

### AV 86

R= PPG n=7 MW-690

Was prepared as above from 0.22 gr AV 57 to give 0.25 gr ,47% yield.

### AV 87

### R= PPG n=17 MW-1270

Was prepared as above from 0.2 gr AV 57 to give 0.33 gr ,33% yield.

### Compounds Synthesized from Tyrosinol

Compounds Synthesized from Compound 6, AV 58

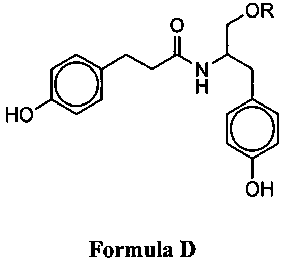

### AV 64

R= PPG n=7 MW-722

Was prepared as above from 0.2 gr **AV 58** to give 0.21 gr, 46% yield.

### AV 65

R= PPG n=17 MW-1302

Was prepared as above from 0.23 gr AV 58 to give 0.28 gr, 29% yield.

### Compounds Synthesized from Compound 8, AV 72

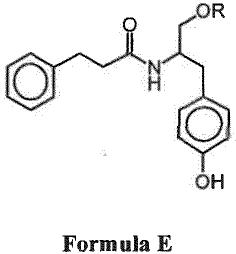

### AV 74S

R= PPG n=7 MW-706

### a. Mesylation of PPG.

106 mg of PPG₄₂₅ (0.25 mmol) was reacted with 90 mole-percent of mesyl chloride (26 mg, 2 drops) and 0.4 mmol pyridine (31.6 mg, 2 drops) to afford the mono-mesylated PPG (A). After combining PPG, mesyl chloride and pyridine, the mesylation reaction was carried out at 0°C over 30 minutes while stirring, and then the reaction was continued for another 60 minutes at room temperature. During mixing, the reaction mixture turned from colorless to milky-white. The mixture was then dissolved in 5 mL methylene chloride and the organic phase washed twice with 1M HCl solution, then twice with 1M NaOH solution and once with water. The organic phase was dried over anhydrous sodium sulfate, filtered and the solvent removed.

### b. Sodium activation.

0.1g of the above product (0.25 mmol) was dissolved in 5 mL of absolute ethanol and then reacted with an equimolar amount of sodium ethoxide in absolute ethanol (previously prepared by reacting 0.25 mg-atom of sodium with an excess of absolute ethanol). The ethanol of the combined solutions was evaporated to total dryness to yield the sodium salt **(B).**

### a. Reacting A and B

A was dissolved in 5 mL of a potassium hydroxide-dried acetonitrile and the solution introduced into a round-bottom flask containing a magnetic stirrer. 5 mL of dried acetonitrile solution of **B** was introduced into the flask, followed by a catalytic amount (few crystals) of potassium iodide. A reflux condenser and a gas bubbler adjusted on top of it were connected to the reaction vessel and the reaction mixture was allowed to reflux under nitrogen atmosphere, while stirring, during 24 hrs. The reaction mixture was then paper-filtered and the solvent removed. The residue was dissolved in 2 mL of ethyl acetate and then passed through a silica-gel column, using ethyl acetate for elution. The TLC (elution with ethyl acetate) UV-absorbing spot at R_{f} = 0.55 turned out to contain the desired product 3 (a mixture of molecules containing different PPG sub-unit lengths), however, containing also some unreacted PPG. Other fractions contained unreacted mesylated PPG and doubly-mesylated PPG.

### AV 78S

R= PPG n=17 MW-1000

Was prepared using the same procedure as **AV 74S,** above, with the substitution of the PPG 7 for PPG 17.

### EXAMPLE 1

The following experiments were conducted to demonstrate the utility of compounds of the invention in treating colon cancer.

Protein tyrosine kinases provide a central switch mechanism in cellular signal transduction pathways, as such they are involved in many cellular processes such as proliferation, metabolism, survival and apoptosis. Several PTKs are known to be activated in cancer cells and drive tumor growth and progression of the disease, Blocking tyrosine kinase activity therefore represents a rational approach to cancer therapy.

Spleen tyrosine kinase was initially found to be expressed only in hemopoietic cells like myeloid cells, macrophages, erythrocytes, platelets and B cells, but has now been found to be expressed in non-hemopoietic cells, and to mediate signaling of various cytokines. As such, Syk is essential for immune system development and function, and for the maintenance of vascular integrity. It has recently been shown that Syk plays an important role in the tumorigenicity of various tumors such as breast, colon, non small-cell lung cancers, as well as the hematologic malignancies such as CML, AML, and B cell lymphoma.

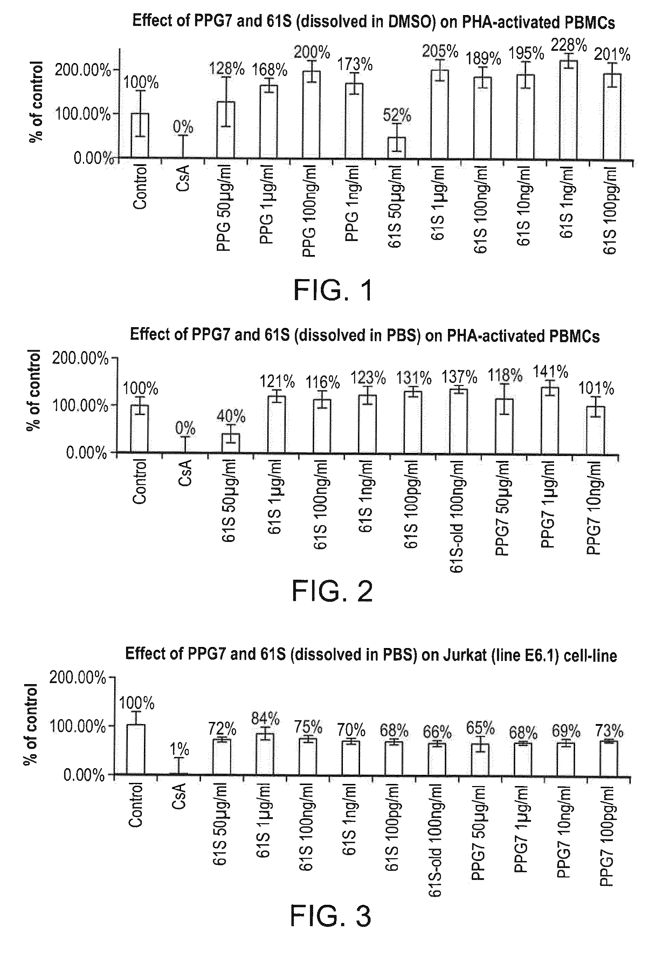

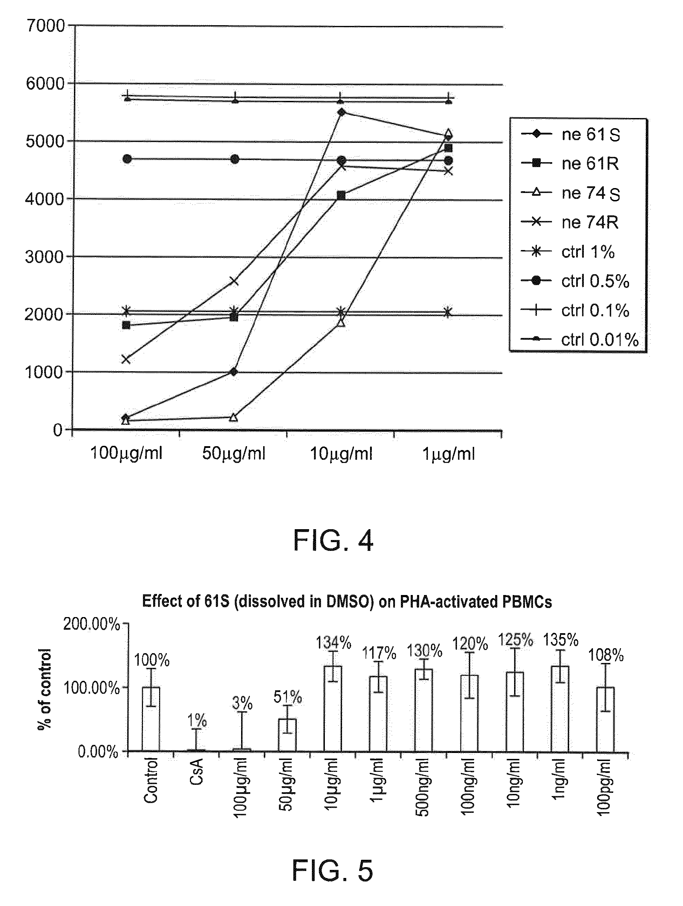

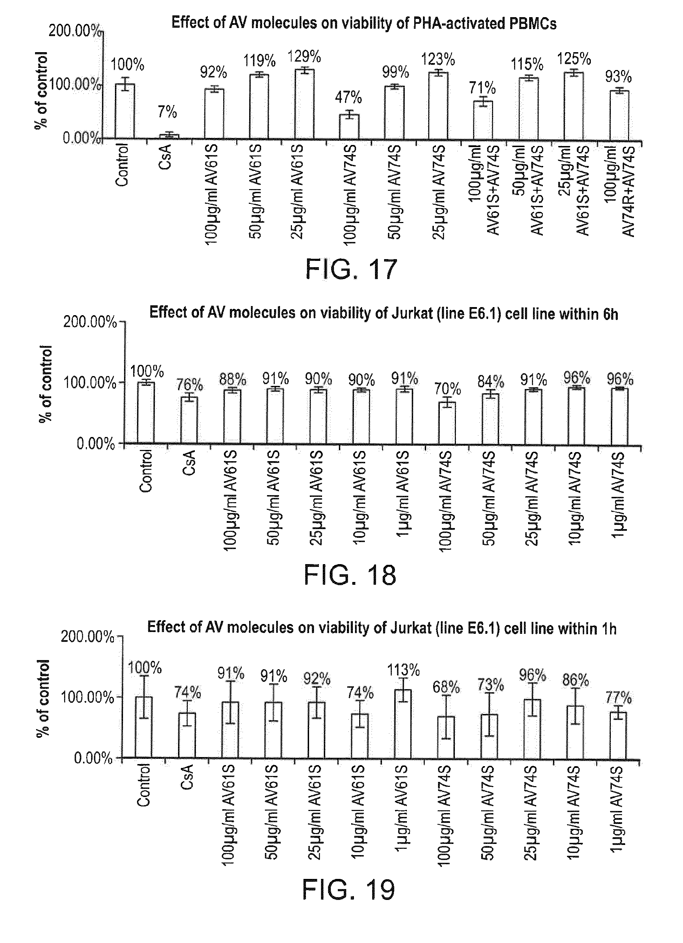

**Fig. 4** shows that Syk-containing Jurkat cells are killed (almost 100%) by AV 74S at a concentration as low as 10µg/ml; by 61S at 50µg/ml; 74R has a killing effect (approximately 85%) at a concentration of 100µg/ml and a lesser effect at 50µg/ml; AV 61R has a killing effect at ∼70% with both 100µg/ml and 50µg/ml concentrations.The jurkat E6.1 line is a Syk-deficient line; in fact, AV 61 S after 7 days has no effect on this cell line (*i.e.,* neither apoptosis nor inhibition, see **Fig. 3****).** Moreover, these cells are viable, as can be seen from Figs. 18 and 19, and both compounds (AV 61 S and AV 74S) do not kill them. The viability study has been performed after 24 and 48 hours of incubation, each time for 3 evaluations; within 1, 3 and 6 hours. **Fig 18** shows the results after 24h of incubation within 6 hours **Fig. 19** shows the results after 48h and within 1 hour after the incubation. These experiments demonstrate that when Syk is present in a transformed cell *(e.g.,* a T-cell leukemia Jurkat cell), AV compounds kill the cell. AV compounds have no killing effect on normal cells (as are the PBMCs, taken always from different donors. The different lymphocyte-proliferation bio-assays have been performed at least four (4) times from different donors, but rather a cell-cycle arrest (probably before the S-phase, as the thymidine incorporation in the cell cycle occurs at the S-phase). When in the transformed cell the Syk is missing, AV compounds have no effect at all, not even an inhibitory effect.

Two other experiments confirmed this observation. First, HL 60 cells (a human promyelocytic leukemia cell line) contains Syk, while CCRF cells, a T cell leukemia cell line - which contain no tyrosine kinase proteins - were treated with compounds AV 60S and AV 78S of the invention over a four day period. The data are shown in Table 1, where AV 60S and AV 78S are seen inhibit HL-60 cells, whereas they appear to have no effect on the CCRF cell line.

Secondly, the effect of compounds of the invention may be compared to the natural inhibitor of Syk, piceatannol. As the data show in Table 2, piceatannol at least partially blocks the inhibitory effect of AV 74S when present in a concentration of 100µg/ml, whereas 74S blocks the apoptotic effect of 50µg/ml and 30µM/ml piceatannol.

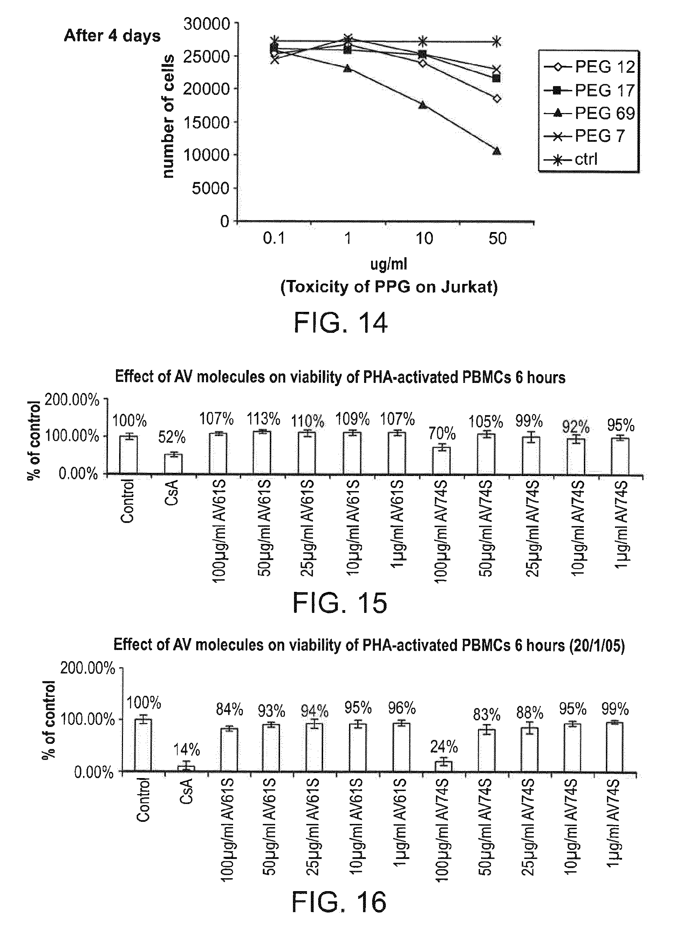

Without wishing to be limited to the following, it appears that compounds of the invention act (inhibit Syk tyrosine kinase-mediated conditions) by blocking of cell growth (i.e., cell cycle arrest) instead of by killing the cells (apoptosis.) FIGs 15-19 illustrate experiments conducted in this regard, using cyclosporine (CsA) as a comparison. In comparison, and as seen in FIGs 18 and 19, this effect is not significantly seen in Jurkat cells.

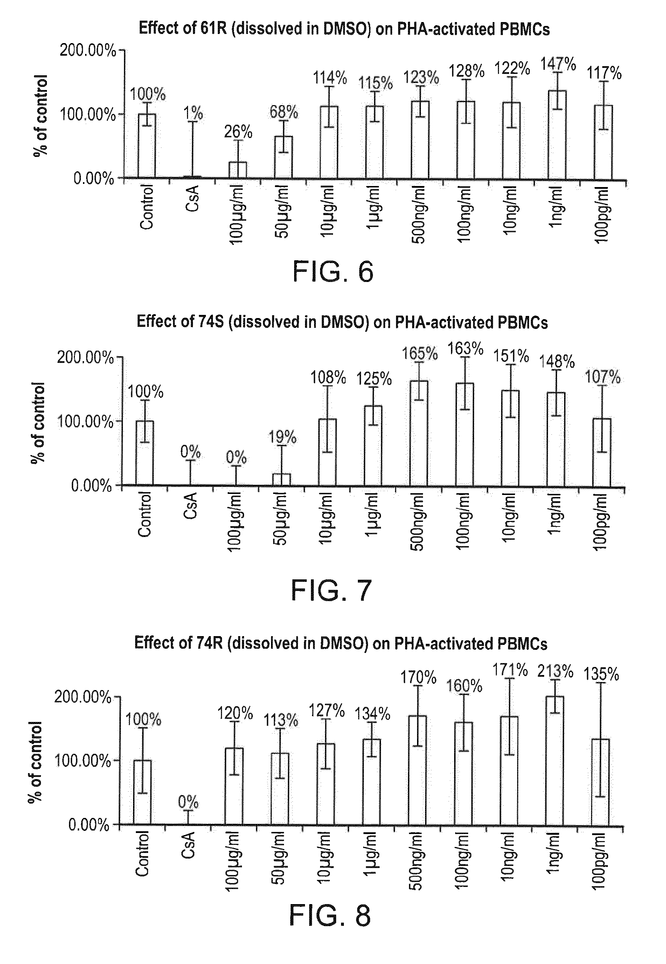

The efficacy of the compounds of the invention is particularly demonstrated with reference to FIGs 5-8. FIG 5 shows the effect of treating PHA-activated PBMCs with varying concentrations of a compound of the invention, AV 61S, whereas FIG 6 shows the effect of treating PHA-activated PBMCs with varying concentrations of AV 61R. FIG 7 shows the effect of treating PHA-activated PBMCs with varying concentrations of a compound of the invention, AV 74S, whereas FIG 8 shows the effect of treating PHA-activated PBMCs with varying concentrations of AV 74R. As can be seen in the above figures, the S- form of the respective compounds (*i.e.,* compounds of the invention) are far better inhibitors of PBMC cells than their R- counterparts.

FIGs 1 and 2 show the effect of diluent on compounds of the invention. FIG 1 shows the inhibitory effect of treating PHA-activated PBMCs with varying concentrations of a compound of the invention, AV 61S in a DMSO carrier, whereas FIG 2 shows a lesser inhibitory effect of the same compound in a PBS carrier.

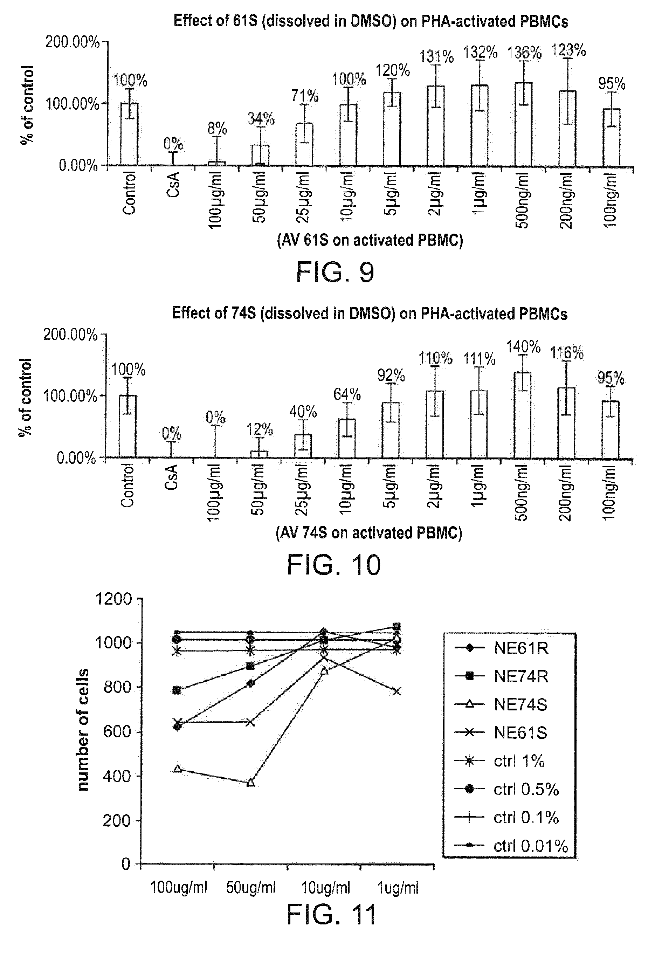

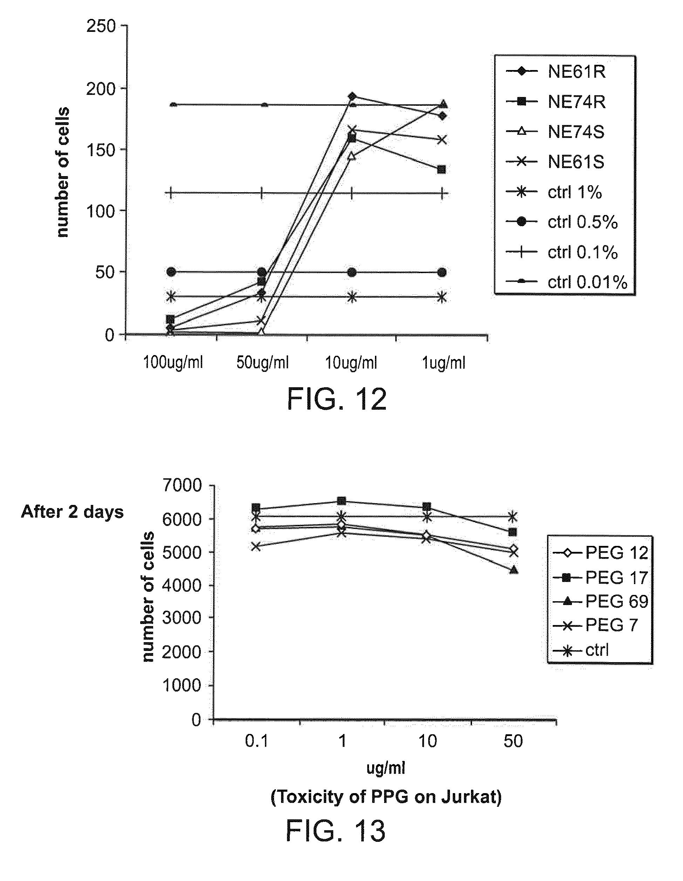

FIGs 11 and 12 compare the effect of compounds of the invention - AV 61S and AV 74S - on human PBMC T and B cells (FIG 11) versus human PBMC monocytes (FIG 12). The figures demonstrate that the effect of the compounds of the invention is more profound on monocytes (which are Syk-containing) as compared to T and B cells. T cells contain ZAP-70, rather than Syk, but B lymphocytes do contain Syk. PPG 7 and PPG 17 (*i.e.,* not AV compounds of the invention) do not appear to be inhibitory. As such, the effect seen on the B and T cells is believed to be due to the activity on the B cells, not the T cells.

Suitable concentrations for administration to obtain an inhibitory effect range between about 20µg /ml and 100µg/ml.

Compounds of the invention therefore can be used to treat tyrosine kinase (Syk)-dependent tumors, and other diseases in which Syk is involved.

**Table 1.**

| **Effect of AV 78S and AV 60S on viability of HL-60 cells after 4 days - XTT method (% of control)** | | | | |
|---|---|---|---|---|
| | **HL 60 (1) %** | **HL 60 (2) %** | **CCRF (1) %** | **CCRF (2) %** |
| 60s (1µg/ml) | 88 | 86 | 100 | 98 |
| 60s (3µg/ml) | 94 | 69 | 89 | 100 |
| 60s (10µg/ml) | 71 | 65 | 94 | 96 |
| 60s (30µg/ml) | 40 | 36 | 92 | 80 |
| 60s (50µg/ml) | 43 | 31 | 83 | 79 |
| 78s (1µg/ml) | 95 | 75 | 94 | 97 |
| 78s (3µg/ml) | 88 | 68 | 80 | 94 |
| 78s (10µg/ml) | | 66 | 88 | 69 |
| 78s (30µg/ml) | 41 | 24 | 75 | 79 |
| 78s (50µg/ml) | 4 | 4 | 53 | 23 |
| PPG (1µg/ml) | | 84 | 97 | 100 |
| PPG (3µg/ml) | 100 | 71 | 92 | 83 |
| PPG (10µg/ml) | 90 | 47 | 98 | 100 |
| PPG (30µg/ml) | 80 | 52 | 96 | 100 |
| PPG (50µg/ml) | 52 | 43 | 84 | 95 |

| | | | | |
|---|---|---|---|---|
| 1. HL-60 cells at a concentration of 3x10⁵ have been tested at the presence and absence of the AV molecules for 4 days. 2. The concentration of DMSO in the system was 0.1% 3. At the end of the incubation period the viability of the cells was determined by the XTT method and read by the ELISA reader | | | | |

**Table 2.**

| **Effect of AV 74S and AV 61S on viability of HL-60 cells after 3 days XTT method (% of control)** | | | | | |
|---|---|---|---|---|---|
| | control | 74 (50µg/ml) | 74 (100µg/ml) | 61 (50µg/ml) | 61 (100µg/ml) |
| Control | 100 | 58 | 17 | 81 | 36 |
| Pic 10µM | 89 | 71 | 56 | 97 | 59 |
| Pic 30µM | 79 | 82 | 31 | 83 | 23 |
| | | | | | |

| | | | XTT - viability | | |
|---|---|---|---|---|---|
| | Control | | 100% | | |
| | PPG 10 µg/ml | | 100% | | |
| | PPG 30 µg/ml | | 100% | | |
| | PPG 50µg/ml | | 84% | | |
| | PPG 100µg/ml | | 56% | | |
| | | | | | |

| **Apoptosis of HL-60 cells after 2 days** | | | | | |
|---|---|---|---|---|---|
| | Control | 74S (50µg/ml) | 74S (100µg/ml) | 74S (50µg/ml) | 74S (100µg/ml) |
| Control | 3% | 15% | 22% | 6% | 7% |
| Pic. 10µM | 20% | 5% | 15% | 9% | 1% |
| Pic. 30µM | 33% | 3% | 20% | 13% | 26% |
| | | | | | |

| | | | Viability Trypan blue | % apoptosis | |
|---|---|---|---|---|---|
| | Control | | 100% | 3% | |
| | PPG 10µg/ml | | ND | ND | |
| | PPG 30µg/ml | | | | |
| | PPG 50µg/ml | | 69% | 5% | |
| | PPG 100µg/ml | | 70% | 7% | |

| | | | | | |
|---|---|---|---|---|---|
| Pic. : Piceatannol ND: Not Determined | | | | | |

### EXAMPLE 2

Compound AV 74S was tested against nine different panels of cell lines according to the NCI *in vitro* anti-cancer drug discovery screen (see Chapter 3, "The NCI Human Tumor Cell Line (60-Cell) Screen: Concept, Implementation and Applications", by Michael R. Boyd in Part I of "Anticancer Drug Development Guide: Preclinical Screening, Clinical Trials and Approval (2nd edition)", edited by B. Teicher, Humana Press, Totowa, New Jersey, 2004). The nine panels consisted of the following respective cell lines:

| Leukaemia | Non-small cell lung cancer | Colon Cancer |
|---|---|---|
| CCRF-CEM | EKVX | COLO 205 |
| HL-60(TB) | HOP-62 | HTC-2998 |
| MOLT-4 | HOP-92 | HCT-16 |
| RPMI-8226 | NCI-H226 | HCT-15 |
| SR | NCIH23 | HT29 |
| | NCI-H322M | KM12 |
| | NCI-H460 | SW-620 |
| | NCI-H522 | |

| CNS cancer | Melanoma | Ovarian cancer |
|---|---|---|
| SF-268 | LOX IMVI | IGROV1 |
| SS-295 | MALME-3M | OVCAR-3 |
| SS-539 | M14 | OVCAR-4 |
| SNB-19 | SK-MEL-2 | OVCAR-5 |
| SNB-75 | SK-MEL-28 | OVCAR-8 |
| U251 | SK-MEL-5 | SK-OV-3 |
| | UACC-257 | |
| | UACC-62 | |

| Renal cancer | Prostate cancer | Breast cancer |
|---|---|---|
| 786-0 | PC-3 | MCF7 |
| A498 | DU-145 | NCI/ADR-RES |
| ACHN | | MDA-MB-231/ATCC |
| CAKI-1 | | HS 578T |
| RXF 393 | | MDA-MB-435 |
| SN12C | | BT-549 |
| TK-10 | | T-47D |
| UO-31 | | |

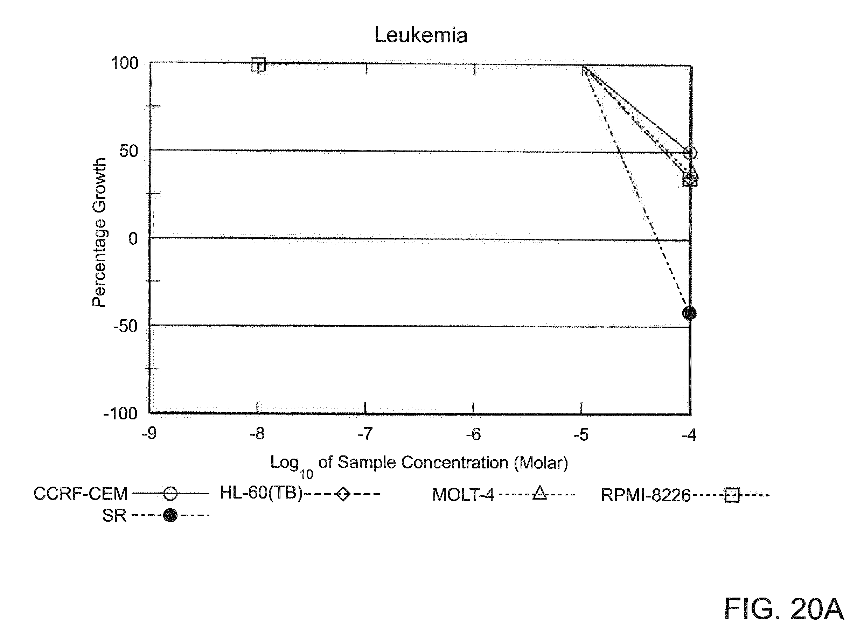

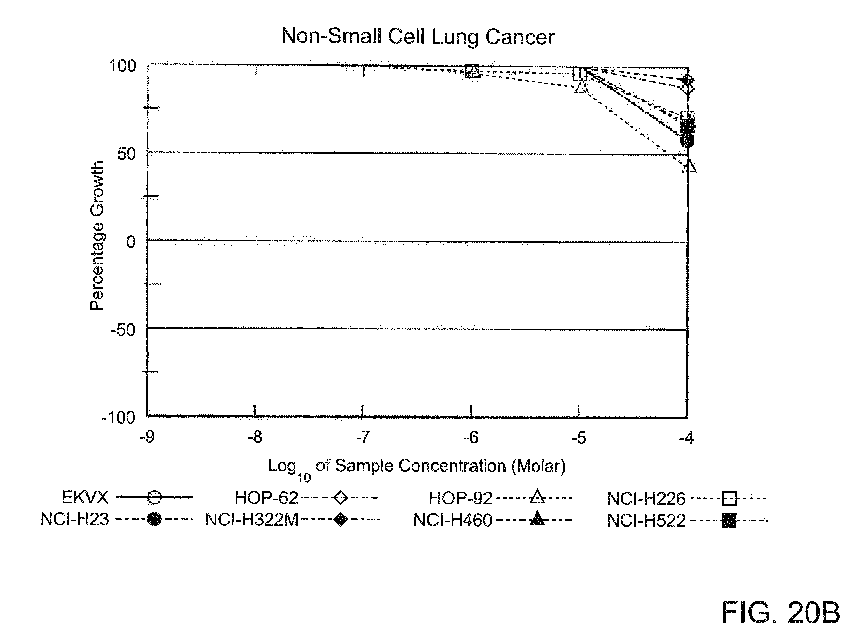

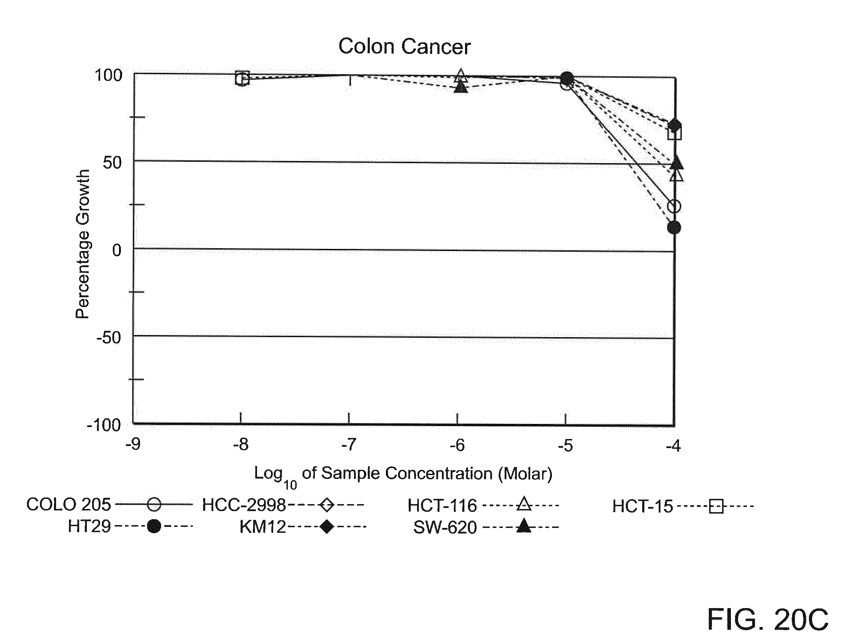

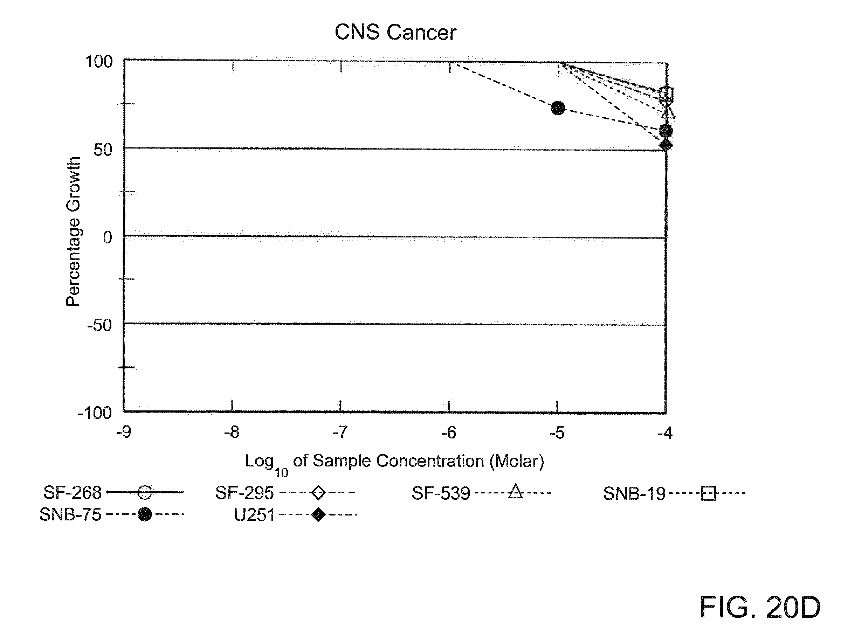

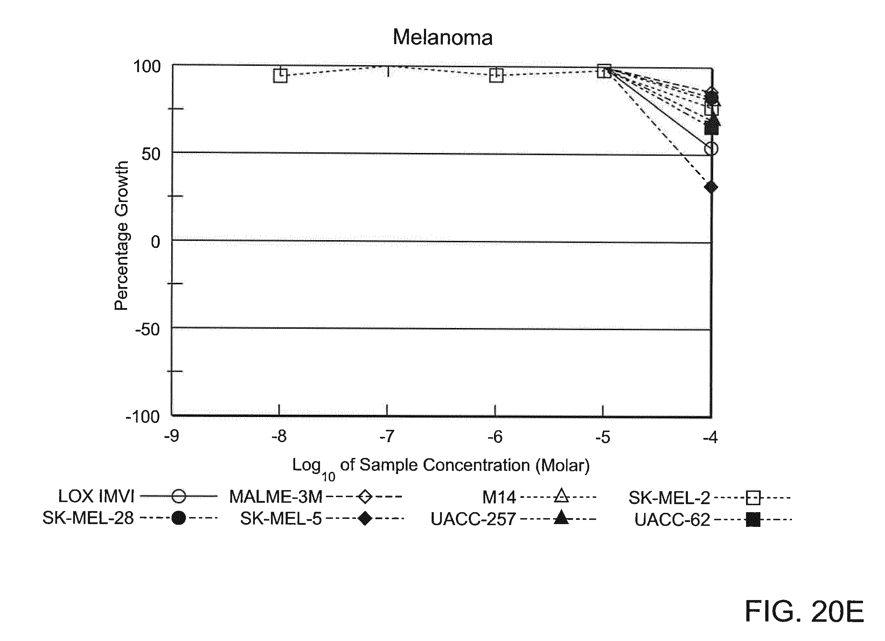

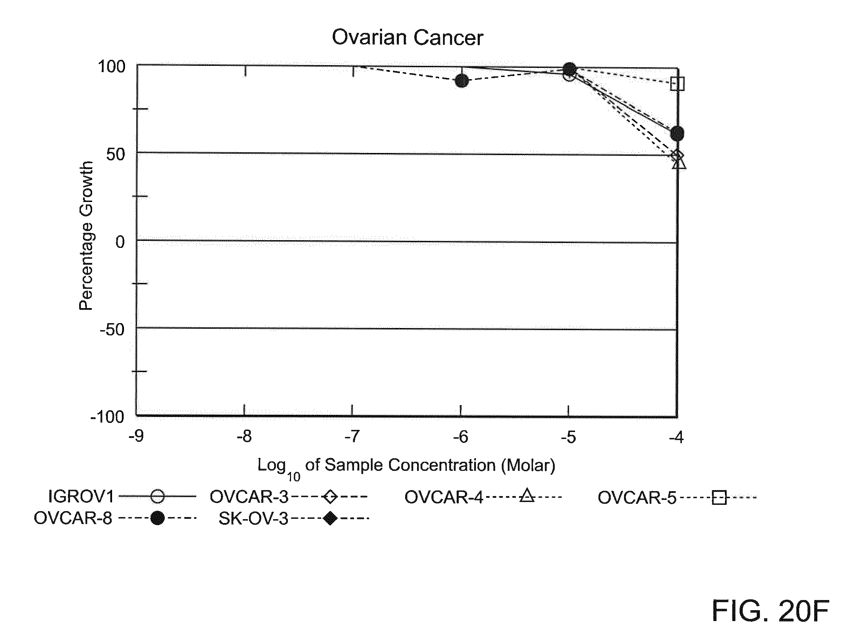

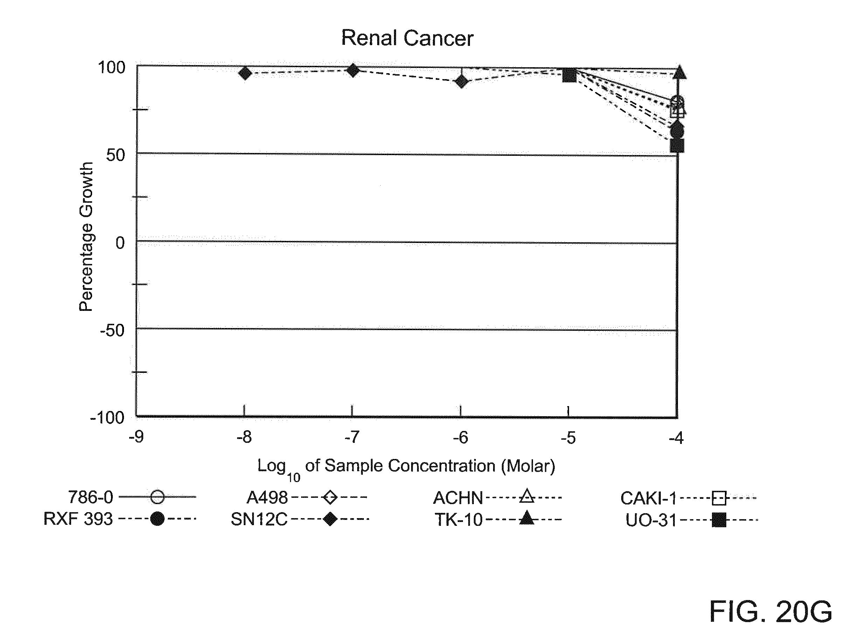

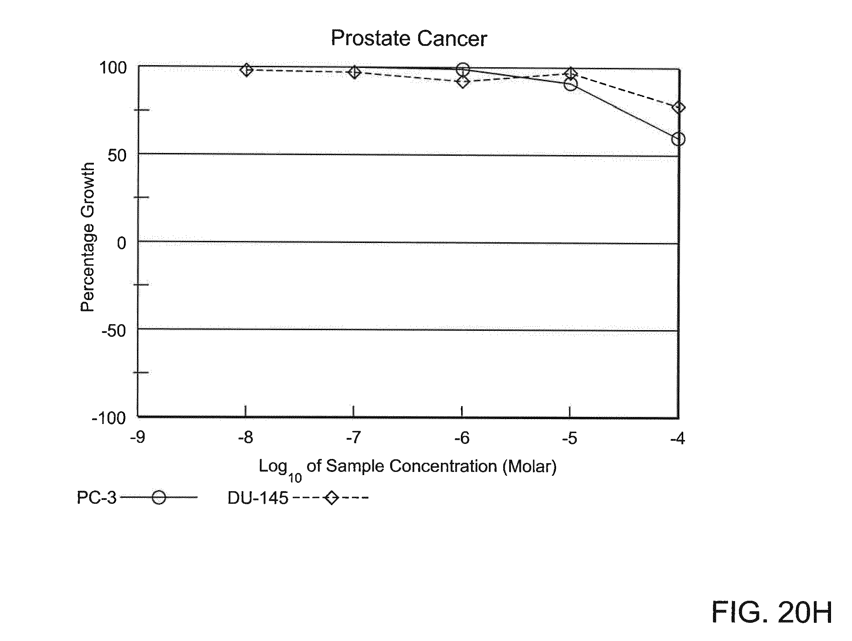

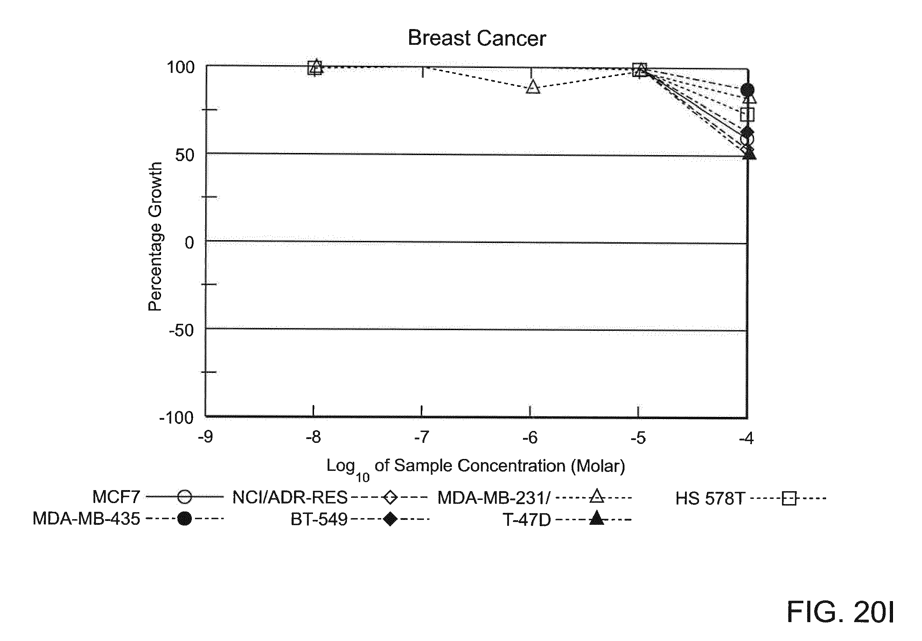

The panel lines were inoculated onto a series of standard 96-well microtitre plates on day 0 at about 20,000 cells/well, and then preincubated in the absence of the compound for 24 hours. AV 74S was then added in five, 10-fold dilutions starting from a log₁₀ concentration of -4.0 (corresponding to 70.6 µg/ml or 100 µmol/ml) and incubated for a further 48 hours. Following this, the cells were *fixed in situ,* washed and dried. Sulforhodamine B (SRB) was added, followed by further washing and drying of the stained, adherent cell mass. The bound stain was solubilised and measured spectrophotometrically. The results are shown in Table 3 and illustrated graphically in FIGS. 20A-I of the accompanying drawings.

**Table 3**

| Log 10 Concentration | | | | | | | | | | | | | | | |
|---|---|---|---|---|---|---|---|---|---|---|---|---|---|---|---|
| | **Time** | | **Mean Optical Densities** | | | | | **Percent Growth** | | | | | | | |
| **Panel/Cell Line** | **Zero** | **Ctrl** | **-8.0** | **-7.0** | **-6.0** | **-5.0** | **-4.0** | **-8.0** | **-7.0** | **-6.0** | **-5.0** | **-4.0** | **G150** | **TGI** | **LC50** |
| Leukemia | | | | | | | | | | | | | | | |
| CCRF-CEM | 0.141 | 0.738 | 0.771 | 0.794 | 0.797 | 0.796 I | 0.437 | 106 | 109 | 110 | 110 | 50 | 9.83E-5 | > 1.00E-4 | >1.00E-4 |
| HL-60(TB) | 0.468 | 1.443 | 1.616 | 1.690 | 1.643 | 1.822 | 0.814 | 118 | 125 | 120 | 139 | 35 | 7.23E-5 | > 1.00E-4 | > 1.00E-4 |
| MOLT-4 | 0.473 | 1.363 | 1.435 | 1.502 | 1.521 | 1.656 | 0.814 | 108 | 116 | 118 | 133 | 38 | 7.52E-5 | > 1.00E-4 | > 1.00E4 |
| RPMI-8226 | 0.404 | 1.301 | 1.292 | 1.393 | 1.414 | 1.399 | 0.719 | 99 | 110 | 113 | 111 | 35 | 6.35E-5 | > 1.00E-4 | > 1.00E-4 |
| SR | 0.494 | 1.317 | 1.375 | 1.473 | 1.482 | 1.507 | 0.286 | 107 | 119 | 120 | 123 | -42 | 2.77E-5 | 5.56E-5 | > 1.00E-4 |
| Non-Small Cell Lung Cancer | | | | | | | | | | | | | | | |
| EKVX | 0.474 | 1.656 | 1.745 | 1.775 | 1.742 | 1.744 | 1.157 | 108 | 110 | 107 | 107 | 58 | > 1.00E-4 | > 1.00E-4 | > 1.00E-4 |
| HOP-62 | 0.404 | 1.104 | 1.128 | 1.174 | 1.183 | 1.224 | 1.018 | 103 | 110 | 111 | 117 | 88 | > 1.00E-4 | > 1.00E-4 | > 1.00E-4 |
| HOP-92 | 0.752 | 1.198 | 1.230 | 1.213 | 1.179 | 1.145 | 0.946 | 107 | 103 | 96 | 88 | 43 | 7.14E-5 | > 1.00E-4 | > 1.00-4 |
| NCI-H226 | 0.619 | 1.178 | 1.194 | 1.213 | 1.161 | 1.157 | 1.014 | 103 | 106 | 97 | 96 | 71 | E 1.00E-4 | > 1.00E-4 | > 1.00E-4 |
| NCI-H23 | 0.511 | 1.458 | 1.489 | 1.560 | 1.550 | 1.568 | 1.069 | 103 | 111 | 110 | 112 | 59 | > 1.00E-4 | > 1.00E-4 | > 1.00E-4 |
| NCI-H322M | 0.628 | 1.594 | 1.646 | 1.659 | 1.684 | 1.687 | 1.527 | 105 | 107 | 109 | 110 | 93 | > 1.00E-4 | > 1.00E-4 | > 1.00E-4 |
| NCI-H460 | 0.270 | 2.135 | 2.208 | 2.272 | 2.180 | 2.278 | 1.547 | 104 | 107 | 102 | 108 | 68 | > 1.00E-4 | > 1.00E-4 | > 1.00E-4 |
| NCI-H522 | 0.779 | 1.924 | 1.948 | 2.024 | 1.983 | 1.938 | 1.546 | 102 | 109 | 105 | 101 | 67 | > 1.00E-4 | > 1.00E-4 | > 1.00E-4 |
| Colon Cancer | | | | | | | | | | | | | | | |
| COLO205 | 0.239 | 1.175 | 1.146 | 1.225 | 1.237 | 1.136 | 0.483 | 97 | 105 | 107 | 96 | 26 | 4.54E-5 | > 1.00E-4 | > 1.00E-4 |
| HCC-2998 | 0.451 | 1.573 | 1.579 | 1.703 | 1.882 | 1.660 | 1.261 | 101 | 112 | 128 | 108 | 72 | > 1.00E-4 | > 1.00E-4 | > 1.00E-4 |
| HCT-116 | 0.195 | 1.715 | 1.730 | 1.763 | 1.703 | 1.827 | 0.855 | 101 | 103 | 99 | 107 | 43 | 7.89E-5 | > 1.00E-4 | > 1.00E-4 |
| HCT-15 | 0.523 | 2.507 | 2.470 | 2.514 | 2.555 | 2.619 | 1.877 | 98 | 100 | 102 | 106 | 68 | > 1.00E-4 | > 1.00E-4 | > 1.00E-4 |
| HT29 | 0.246 | 1.610 | 1.630 | 1.677 | 1.674 | 1.601 | 0.439 | 101 | 105 | 105 | 99 | 14 | 3.79E-5 | > 1.00E-4 | > 1.00E-4 |
| KM12 | 0.339 | 1.354 | 1.439 | 1.418 | 1.406 | 1.409 | 1.075 | 108 | 106 | 105 | 105 | 73 | > 1.00E-4 | > 1.00E-4 | > 1.00E-4 |
| SW-620 | 0.131 | 1.023 | 1.023 | 1.064 | 0.957 | 1.032 | 0.579 | 100 | 105 | 93 | 101 | 50 | > 1.00E-4 | > 1.00E-4 | > 1.00E-4 |
| CNS Cancer | | | | | | | | | | | | | | | |
| SF-268 | 0.346 | 1.126 | 1.174 | 1.210 | 1.199 | 1.299 | 0.996 | 106 | 111 | 109 | 113 | 83 | > 1.00E-4 | > 1.00E-4 | > 1.00E-4 |
| SF-295 | 0.764 | 1.914 | 2.002 | 2.013 | 1.990 | 1.994 | 1.663 | 108 | 109 | 107 | 107 | 78 | > 1.00E-4 | > 1.00E-4 | > 1.00E-4 |
| SF-539 | 0.336 | 1.275 | 1.331 | 1.367 | 1.290 | 1.308 | 1.000 | 106 | 110 | 102 | 104 | 71 | > 1.00E-4 | > 1.00E-4 | > 1.00E-4 |
| SNB-19 | 0.318 | 1.258 | 1.307 | 1.348 | 1.325 | 1.338 | 1.088 | 105 | 110 | 107 | 109 | 82 | > 1.00E-4 | > 1.00E-4 | > 1.00E-4 |
| SNB-75 | 0.652 | 1.102 | 1.129 | 1.137 | 1.116 | 0.987 | 0.929 | 106 | 108 | 103 | 74 | 61 | > 1.00E-4 | > 1.00E-4 | > 1.00E-4 |
| U251 | 0.218 | 1.342 | 1.390 | 1.437 | 1.382 | 1.367 | 0.819 | 104 | 108 | 104 | 102 | 53 | > 1.00E-4 | > 1.00E-4 | > 1.00E-4 |
| Melanoma | | | | | | | | | | | | | | | |
| LOX IMVI | 0.248 | 1.656 | 1.830 | 1.772 | 1.757 | 1.705 | 1.007 | 112 | 108 | 107 | 103 | 54 | > 1.00E-4 | > 1.00E-4 | > 1.00E-4 |
| MALME-3M | 0.499 | 0.992 | 1.022 | 1.021 | 1.018 | 1.024 | 0.922 | 106 | 106 | 105 | 106 | 86 | > 1.00E-4 | > 1.00E-4 | > 1.00E-4 |
| M14 | 0.434 | 1.668 | 1.744 | 1.727 | 1.724 | 1.800 | 1.434 | 106 | 105 | 105 | 111 | 81 | > 1.00E-4 | > 1.00E-4 | > 1.00E-4 |
| SK-MEL-2 | 0.337 | 0.878 | 0.847 | 0.924 | 0.850 | 0.868 | 0.755 | 94 | 109 | 95 | 98 | 77 | > 1.00E-4 | > 1.00E-4 | > 1.00E-4 |
| SK-MEL-28 | 0.394 | 1.397 | 1.428 | 1.397 | 1.409 | 1.431 | 1.228 | 103 | 100 | 101 | 103 | 83 | > 1.00E-4 | > 1.00E-4 | > 1.00E-4 |
| SK-MEL-5 | 0.246 | 2.136 | 2.202 | 2.235 | 2.191 | 2.168 | 0.843 | 104 | 105 | 103 | 102 | 32 | 5.46E-5 | > 1.00E-4 | > 1.00E-4 |
| UACC-257 | 0.777 | 1.991 | 2.075 | 2.071 | 2.092 | 2.056 | 1.631 | 107 | 107 | 108 | 105 | 70 | > 1.00E-4 | > 1.00E-4 | > 1.00E-4 |
| UACC-62 | 0.513 | 2.111 | 2.241 | 2.264 | 2.284 | 2.188 | 1.575 | 108 | 110 | 111 | 105 | 66 | > 1.00E-4 | > 1.00E4 | > 1.00E-4 |
| Ovarian Cancer | | | | | | | | | | | | | | | |
| IGROV1 | 0.183 | 0.895 | 1.019 | 0.949 | 0.917 | 0.869 | 0.623 | 117 | 108 | 103 | 96 | 62 | > 1.00E-4 | > 1.00E-4 | > 1.00E-4 |
| OVCAR-3 | 0.538 | 1.620 | 1.683 | 1.689 | 1.709 | 1.736 | 1.083 | 106 | 106 | 108 | 111 | 50 | > 1.00E-4 | > 1.00E-4 | > 1.00E-4 |
| OVCAR-4 | 0.443 | 1.219 | 1.288 | 1.301 | 1.297 | 1.293 | 0.796 | 109 | 111 | 110 | 110 | 45 | 8.49E-5 | > 1.00E-4 | > 1.00E-4 |
| OVCAR-5 | 0.346 | 0.82 | u.903 | 0.935 | 0.942 | 0.952 | 0.845 | 102 | 108 | 109 | 111 | 91 | > 1.00E-4 | > 1.00E-4 | > 1.00E-4 |
| OVCAR-8 | 0.315 | 1.583 | 1.607 | 1.654 | 1.476 | 1.567 | 1.109 | 102 | 106 | 92 | 99 | 63 | > 1.00E-4 | > 1.00E-4 | > 1.00E-4 |
| SK-OV-3 | 0.442 | 1.004 | 1.029 | 1.100 | 1.052 | 1.055 | 1.005 | 104 | 117 | 109 | 109 | 100 | > 1.00E-4 | > 1.00E-4 | > 1.00E-4 |
| Renal Cancer | | | | | | | | | | | | | | | |
| 786-0 | 0.303 | 1.385 | 1.449 | 1.478 | 1.513 | 1.629 | 1.183 | 106 | 109 | 112 | 123 | 81 | > 1.00E-4 | > 1.00E-4 | > 1.00E-4 |
| A498 | 0.852 | 1.614 | 1.632 | 1.721 | 1.699 | 1.708 | 1.472 | 102 | 114 | 111 | 112 | 81 | > 1.00E-4 | > 1.00E-4 | > 1.00E-4 |
| ACHN | 0.335 | 1.357 | 1.420 | 1.476 | 1.462 | 1.395 | 1.123 | 106 | 112 | 110 | 104 | 77 | > 1.00E-4 | > 1.00E-4 | > 1.00E-4 |
| CAKI-1 | 0.542 | 0.876 | 0.886 | 0.886 | 0.893 | 0.912 | 0.795 | 103 | 103 | 105 | 111 | 76 | > 1.00E-4 | > 1.00E-4 | > 1.00E-4 |
| RXF 393 | 0.835 | 1.579 | 1.639 | 1.678 | 1.591 | 1.588 | 1.309 | 108 | 113 | 102 | 101 | 64 | > 1.00E-4 | > 1.00E-4 | > 1.00E-4 |
| SN12C | 0.506 | 1.268 | 1.239 | 1.252 | 1.209 | 1.266 | 1.017 | 96 | 98 | 92 | 100 | 67 | > 1.00E-4 | > 1.00E-4 | > 1.00E-4 |
| TK-10 | 0.484 | 1.124 | 1.213 | 1.245 | 1.240 | 1.265 | 1.107 | 114 | 119 | 118 | 122 | 97 | > 1.00E-4 | > 1.00E-4 | > 1.00E-4 |
| UO-31 | 0.482 | 1.687 | 1.685 | 1.818 | 1.716 | 1.645 | 1.151 | 100 | 111 | 102 | 96 | 56 | > 1.00E-4 | > 1.00E-4 | > 1.00E-4 |
| Prostate Cancer | | | | | | | | | | | | | | | |
| PC-3 | 0.306 | 1.262 | 1.283 | 1.298 | 1.249 | 1.177 | 0.879 | 102 | 104 | 99 | 91 | 60 | > 1.00E-4 | > 1.00E-4 | > 1.00E-4 |
| DU-145 | 0.134 | 0.771 | 0.757 | 0.751 | 0.721 | 0.749 | 0.632 | 98 | 97 | 92 | 97 | 78 | > 1.00E-4 | > 1.00E-4 | > 1.00E-4 |
| Breast Cancer | | | | | | | | | | | | | | | |
| MCF7 | 0.440 | 2.170 | 2.258 | 2.276 | 2.300 | 2.415 | 1.476 | 105 | 104 | 108 | 114 | 60 | > 1.00E-4 | > 1.00E-4 | > 1.00E-4 |
| NCI/ADR-RES | 0.302 | 0.947 | 0.982 | 1.021 | 0.990 | 0.980 | 0.651 | 105 | 111 | 107 | 105 | 54 | > 1.00E-4 | > 1.00E-4 | > 1.00E-4 |
| MDA-MB-231/ATCC | 0.513 | 1.458 | 1.452 | 1.509 | 1.340 | 1.436 | 1.296 | 99 | 105 | 88 | 98 | 83 | > 1.00E-4 | > 1.00E-4 | > 1.00E-4 |
| HS 578T | 0.513 | 1.270 | 1.261 | 1.284 | 1.267 | 1.263 | 1.074 | 99 | 102 | 100 | 99 | 74 | > 1.00E-4 | > 1.00E-4 | > 1.00E-4 |
| MDA-MB-435 | 0.871 | 2.632 | 2.657 | 2.661 | 2.673 | 2.688 | 2.330 | 108 | 108 | 108 | 109 | 88 | > 1.00E-4 | > 1.00E-4 | > 1.00E-4 |
| BT-549 | 0.606 | 1.391 | 1.423 | 1.468 | 1.406 | 1.445 | 1.108 | 104 | 110 | 102 | 107 | 64 | > 1.00E-4 | > 1.00E-4 | > 1.00E-4 |
| T-47D | 0.632 | 1.678 | 1.782 | 1.795 | 1.763 | 1.702 | 1.171 | 110 | 111 | 108 | 102 | 51 | > 1.00E-4 | > 1.00E-4 | > 1.00E-4 |

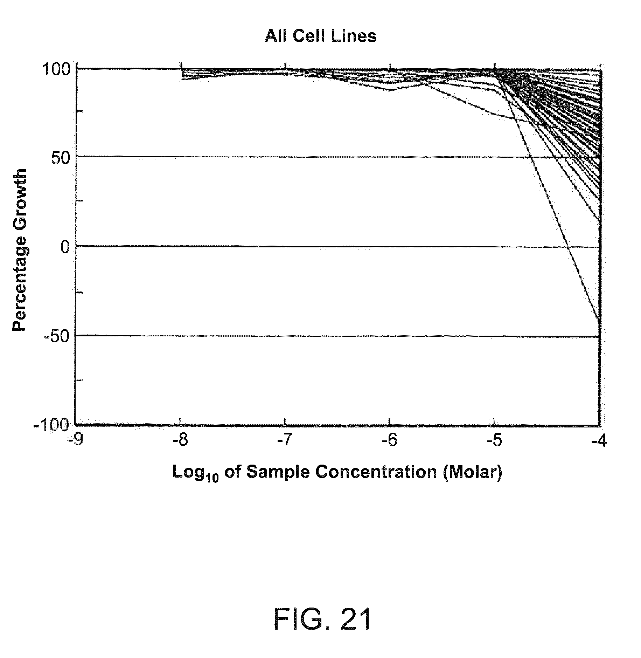

The dose response curves for AV 74S against all cell lines are shown in Fig. 21.

AV 74S showed a log₁₀ GI₅₀ of less than -4.00 for eleven of the cell lines tested.

## Claims

1. A compound for use in treating a cell proliferative disorder in a subject, said compound being the S-enantiomer of formula I: 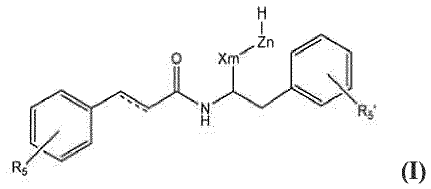 wherein:
the dotted line represents a single or a double bond;
R₅ and R₅' are independently H or OH;
X is -CH₂O, -CH₂CH₂O, -CH(CH₃)CH₂O, or -CH₂CH(CH₃)O;
Z is -CH₂CH₂O, -CH(CH₃)CH₂O, or -CH₂CH(CH₃)O;
m is 1; and
n is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50;
or a pharmaceutically effective salt thereof wherein the cell proliferative disorder is colon cancer.

2. A compound for use according to claim 1, where Z is -CH₂CH₂O.

3. A compound for use according to claim 1, where one of R₅ and R₅' is -OH and the other of R₅ and R₅' is -H.

4. A compound for use according to claim 1, where both of R₅ and R₅' are -OH.

5. A compound for use according to claim 1, where X is -CH₂O.

6. The compound for use according to claim 1, where Z is -CH(CH₃)CH₂O.

7. A compound for use according to claim 1, wherein m is 1.

8. A compound for use according to claim 1, wherein n is 1, 2 or 7.

9. A compound for use according to claim 1, wherein the compound is the S-enantiomer of the following formula (II) wherein R is PPG and n is 7

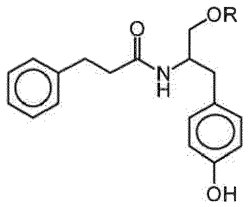

10. A pharmaceutical composition for use in treating a cell proliferative disorder comprising a compound as claimed in any of claims 1-9 and one or more pharmaceutically acceptable excipients, wherein the cell proliferative disorder comprises colon cancer.

11. Use of a compound as claimed in any of claims 1 to 9 in the manufacture of a medicament for the treatment of a cell proliferative disorder comprising colon cancer. 17715365v.1

## Patentansprüche

1. Verbindung zur Verwendung bei der Behandlung einer Störung der Zellproliferation bei einem Individuum, wobei die Verbindung das S-Enantiomer der Formel I ist: 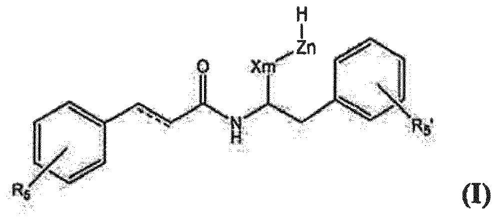 worin:
die gestrichelte Linie eine Einfach- oder eine Doppelbindung darstellt;
R₅ und R₅' unabhängig H oder OH sind;
X -CH₂O, -CH₂CH₂O, -CH(CH₃)CH₂O oder -CH₂CH(CH₃)O ist;
Z -CH₂CH₂O, -CH(CH₃)CH₂O oder -CH₂CH(CH₃)O ist;
m 1 ist;
n 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 oder 50 ist;
oder ein pharmazeutisch wirksames Salz davon, wobei die Störung der Zellproliferation Darmkrebs ist.

2. Verbindung zur Verwendung nach Anspruch 1, wobei Z -CH₂CH₂O ist.

3. Verbindung zur Verwendung nach Anspruch 1, wobei eines von R₅ und R₅' -OH ist und das andere von R₅ und R₅' -H ist.

4. Verbindung zur Verwendung nach Anspruch 1, wobei R₅ und R₅' beide -OH sind.

5. Verbindung zur Verwendung nach Anspruch 1, wobei X -CH₂O ist.

6. Verbindung zur Verwendung nach Anspruch 1, wobei Z -CH(CH₃)CH₂O ist.

7. Verbindung zur Verwendung nach Anspruch 1, worin m 1 ist.

8. Verbindung zur Verwendung nach Anspruch 1, worin n 1, 2 oder 7 ist.

9. Verbindung zur Verwendung nach Anspruch 1, wobei die Verbindung das S-Enantiomer der folgenden Formel (II) ist, worin R PPG ist und n 7 ist.

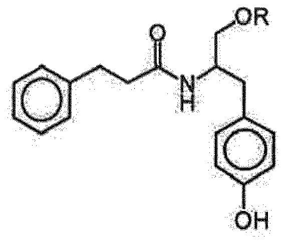

10. Pharmazeutische Zusammensetzung zur Verwendung bei der Behandlung einer Störung der Zellproliferation, umfassend eine Verbindung gemäß einem der Ansprüche 1-9 und ein oder mehr pharmazeutisch verträgliche Hilfsstoffe, wobei die Störung der Zellproliferation Darmkrebs umfasst.

11. Verwendung einer Verbindung gemäß einem der Ansprüche 1 bis 9 zur Herstellung eines Medikaments zur Behandlung einer Störung der Zellproliferation, die Darmkrebs umfasst.

## Revendications

1. Composé pour utilisation dans le traitement d'un trouble de la prolifération cellulaire chez un sujet, ledit composé étant l'énantiomère S de formule I : 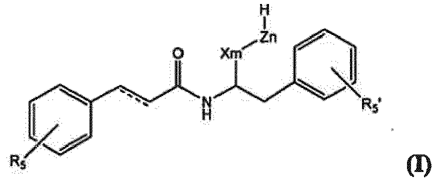 dans laquelle :
la ligne de tirets représente une liaison simple ou double ;
R₅ et R₅' sont indépendamment H ou OH ;
X est -CH₂O, -CH₂CH₂O, -CH(CH₃)CH₂O ou -CH₂CH(CH₃)O ;
z est -CH₂CH₂O, -CH(CH₃)CH₂O ou -CH₂CH(CH₃O ;
m vaut 1 ; et
n vaut 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 ou 50 ;
ou un sel pharmaceutiquement efficace de celui-ci,
dans lequel le trouble de la prolifération cellulaire est un cancer du côlon.

2. Composé pour utilisation selon la revendication 1, dans lequel Z est -CH₂CH₂O.

3. Composé pour utilisation selon la revendication 1, dans lequel l'un de R₅ et R₅' est -OH et l'autre de R₅ et R₅' est -H.

4. Composé pour utilisation selon la revendication 1, dans lequel R₅ et R₅' sont tous deux -OH.

5. Composé pour utilisation selon la revendication 1, dans lequel X est -CH₂O.

6. Composé pour utilisation selon la revendication 1, dans lequel Z est -CH(CH₃)CH₂O.

7. Composé pour utilisation selon la revendication 1, dans lequel m vaut 1.

8. Composé pour utilisation selon la revendication 1, dans lequel n vaut 1, 2 ou 7.

9. Composé pour utilisation selon la revendication 1, lequel composé est l'énantiomère S de la formule (II) suivante où R est PPG et n vaut 7 :

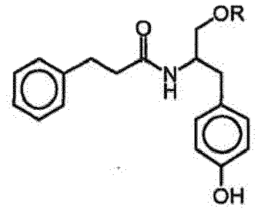

10. Composition pharmaceutique pour utilisation dans le traitement d'un trouble de la prolifération cellulaire, comprenant un composé selon l'une quelconque des revendications 1 à 9 et un ou plusieurs excipient(s) pharmaceutiquement acceptable(s), dans laquelle le trouble de la prolifération cellulaire comprend un cancer du côlon.

11. Utilisation d'un composé selon l'une quelconque des revendications 1 à 9 dans la fabrication d'un médicament destiné au traitement d'un trouble de la prolifération cellulaire, comprenant un cancer du côlon.
